# EUROPEAN PATENT SPECIFICATION

(11) **EP 0 792 278 B1**
(45) Date of publication and mention of the grant of the patent: **13.06.2007**
(21) Application number: 95903521.3
(22) Date of filing: 07.11.1994
(51) Int. Cl.: C07H 17/00, C12P 21/06, C07K 14/00, C12Q 1/68, A61K 31/70, A61K 35/14, C12N 5/00, C12N 15/00

(54) **TUMOR NECROSIS FACTOR-GAMMA**
TUMORNEKROSE-FAKTOR-GAMMA
POLYPEPTIDE GAMMA APPARTENANT A LA FAMILLE DES FACTEURS DE NECROSE TUMORALE (FNT)

(43) Date of publication of application: 03.09.1997
(73) Proprietor: HUMAN GENOME SCIENCES, INC., Rockville, MD 20850-3338 (US)
(72) Inventor: YU, Guo-Liang, Darnestown, MD 20878 (US); NI, Jian, Gaithersburg, MD 20878 (US); ROSEN, Craig A., Laytonsville, MD 20882 (US)
(74) Representative: Vossius & Partner
(86) International application number: PCT/US1994/012880
(87) International publication number: WO 1996/014328

(56) References cited:
- CA-A- 2 114 311
- US-A- 4 677 063
- CANCER COMMUNICATIONS, Vol. 3, No. 1, issued 1991, M.G. ROSENBLUM et al., "Antibody-Mediated Delivery of Tumor Necrosis Factor (TNF-alpha): Improvement of Cytotoxicity and Reduction of Cellular Resistance", pages 21-27.
- ASM NEWS, Vol. 58, No. 9, issued September 1992, J. PENNINGTON, "TNF: Therapeutic Target in Patients with Sepsis", pages 479-482.
- PROC. NATL. ACAD. SCI. U.S.A., Vol. 87, issued November 1990, T. GATANAGA et al., "Purification and Characterization of an Inhibitor (Soluble Tumor Necrosis Factor Receptor) for Tumor Necrosis Factor and Lymphotoxin Obtained from the Serum Ultrafiltrates of Human Cancer Patients", pages 8781-8784.
- NUCLEIC ACIDS RESEARCH, Vol. 13, No. 17, issued 1985, G.E. NEDWIN et al., "Human Lymphotoxin and Tumor Necrosis Factor Genes: Structure, Homology and Chromosomal Localization", pages 6361-6373.
- PROC. NATL. ACAD. SCI. U.S.A., Vol. 86, issued December 1989, D.E. HALLAHAN et al., "Increasing Tumor Necrosis Factor alpha mRNA After Cellular Exposure to Ionizing Radiation", pages 10104-10107.

### SEQUENCE LISTING

(1) GENERAL INFORMATION:
   (i) APPLICANT: YU, ET AL.
   (ii) TITLE OF INVENTION: Human Tumor Necrosis Factor-Gamma
   (iii) NUMBER OF SEQUENCES: 2
   (iv) CORRESPONDENCE ADDRESS:
      (A) ADDRESSEE: CARELLA, BYRNE, BAIN, GILFILLAN, CECCHI, STEWART & OLSTEIN
      (B) STREET: 6 BECKER FARM ROAD
      (C) CITY: ROSELAND
      (D) STATE: NEW JERSEY
      (E) COUNTRY: USA
      (F) ZIP: 07068
   (v) COMPUTER READABLE FORM:
      (A) MEDIUM TYPE: 3.5 INCH DISKETTE
      (B) COMPUTER: IBM PS/2
      (C) OPERATING SYSTEM: MS-DOS
      (D) SOFTWARE: WORD PERFECT 5.1
   (vi) CURRENT APPLICATION DATA:
      (A) APPLICATION NUMBER:
      (B) FILING DATE: Submitted herewith
      (C) CLASSIFICATION:
   (vii) PRIOR APPLICATION DATA
      (A) APPLICATION NUMBER:
      (B) FILING DATE:
   (viii) ATTORNEY/AGENT INFORMATION:
      (A) NAME: FERRARO, GREGORY D.
      (B) REGISTRATION NUMBER: 36,134
      (C) REFERENCE/DOCKET NUMBER: 325800-256
   (ix) TELECOMMUNICATION INFORMATION:
      (A) TELEPHONE: 201-994-1700
      (B) TELEFAX: 201-994-1744
(2) INFORMATION FOR SEQ ID NO:1:
   (i) SEQUENCE CHARACTERISTICS
      (A) LENGTH: 2442 BASE PAIRS
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:1:
   CCCAATCAAG AGAAATTCCA TACTATCACC AGTTGGCCGA CTTTCCAAGT CTAGTGCAGA   60
   AATCCAAGGC ACCTCACACC TAGAGTTCCT ATACCTCTGA GACTCCAGAG GAAAGAACAA  120
   GACAGTGCAG AAGGATATGT TAGAACCCAC TGAAAACCTA GAAGGTTGAA AAGGAAGCAT  180
   ACCCTCCTGA CCTATAAGAA AATTTTCAGT CTGCAGGGGG ATATCCTTGT GGCCCAAGAC  240
   ATTGGTGTTA TCATTTGACT AAGAGGAAAT TATTTGTGGT GAGCTCTGAG TGAGGATTAG  300
   GACCAGGGAG ATGCCAAGTT TCTATCACTT ACCTCATGCC TGTAAGACAA GTGTTTTGTT  360
   CCAATTGATG AATGGGGAGA AAACAGTTCA GCCAATCACT TATGGGCACA GAATGGAATT  420
   TGAAGGGTCT GGTGCCTGCC CTTGTCATAC GTAAACAAGA GAGGCATCGA TGAGTTTTAT  480
   CTGAGTCATT TGGGAAAGGA TAATTCTTGC ACCAAGCCAT TTTCCTAAAC ACAGAAGAAT  540
   AGGGGGATTC CTTAACCTTC ATTGTTCTCC AGGATCATAG GTCTCAGGAT AAATTAAAAA  600
   TTTTCAGGTC AGACCACTCA GTCTCAGAAA GGCAAAGTAA TTTGCCCCAG GTCACTAGTC  660
   CAAGATGTTA TTCTCTTTGA ACAAATGTGT ATGTCCAGTC ACATATTCTT CATTCATTCC  720
   TCCCCAAAGC AGTTTTTAGC TGTTAGGTAT ATTCGATCAC TTTAGTCTAT TTTGAAAATG  780
   ATATGAGACG CTTTTTAAGC AAAGTCTACA GTTTCCCAAT GAGAAAATTA ATCCTCTTTC  840
   TTGTCTTTCC AGTTGTGAGA CAAACTCCCA CACAGCACTT TAAAAATCAG TTCCCAGCTC  900
   TGCACTGGGA ACTAGAACTA GGCCTGGCCT TCACCAAGAA CCGAATGAAC TATACCAACA  960
   AATTCCTGCT GATCCCAGAG TCGGGAGACT ACTTCATTTA CTCCCAGGTC ACATTCCGTG  1020
   GGATGACCTC TGAGTGCAGT GAAATCAGAC AAGCAGGCCG ACCAAACAAG CCAGACTCCA  1080
   TCACTGTGGT CATCACCAAG GTAACAGACA GCTACCCTGA GCCAACCCAG CTCCTCATGG  1140
   GGACCAAGTC TGTATGCGAA GTAGGTAGCA ACTGGTTCCA GCCCATCTAC CTCGGAGCCA  1200
   TGTTCTCCTT GCAAGAAGGG GACAAGCTAA TGGTGAACGT CAGTGACATC TCTTTGGTGG  1260
   ATTACACAAA AGAAGATAAA ACCTTCTTTG GAGCCTTCTT ACTATAGGAG GAGAGCAAAT  1320
   ATCATTATAT GAAAGTCCTC TGCCACCGAG TTCCTAATTT TCTTTGTTCA AATGTAATTA  1380
   TAACCAGGGG TTTTCTTGGG GCCGGGAGTA GGGGGCATTC CACAGGGACA ACGGTTTAGC  1440
   TATGAAATTT GGGGCCAAAA TTTCACACTT CATGTGCCTT ACTGATGAGA GTACTAACTG  1500
   GAAAAAGGCT GAAGAGAGCA AATATATTAT TAAGATGGGT TGGAGGATTG GCGAGTTTCT  1560
   AAATATTAAG ACACTGATCA CTAAATGAAT GGATGATCTA CTCGGGTCAG GATTGAAAGA  1620
   GAAATATTTC AACACCTCCC TGCTATACAA TGGTCACCAG TGGTCCAGTT ATTGTTCAAT  1680
   TTGATCATAA ATTTGCTTCA ATTCAGGAGC TTTGAAGGAA GTCCAAGGAA AGCTCTAGAA  1740
   AACAGTATAA ACTTTCAGAG GCAAAATCCT TCACCAATTT TTCCACATAC TTTCATGCCT  1800
   TGCCTAAAAA AAATGAAAAG AGAGTTGGTA TGTCTCATGA ATGTTCACAC AGAAGGAGTT  1860
   GGTTTTCATG TCATCTACAG CATATGAGAA AAGCTACCTT TCTTTTGATT ATGTACACAG  1920
   ATATCTAAAT AAGGAAGTTT GAGTTTCACA TGTATATCCC AAATACAACA GTTGCTTGTA  1980
   TTCAGTAGAG TTTTCTTGCC CACCTATTTT GTGCTGGGTT CTACCTTAAC CCAGAAGACA  2040
   CTATGAAAAA CAAGACAGAC TCCACTCAAA ATTTATATGA ACACCACTAG ATACTTCCTG  2100
   ATCAAACATC AGTCAACATA CTCTAAAGAA TAACTCCAAG TCTTGGCCAG GCGCAGTGGC  2160
   TCACACCTGT AATCCCAACA CTTTGGGAGG CCAAGGTGGG TGGATCATCT AAGGCCGGGA  2220
   GTTCAAGACC AGCCTGACCA ACGTGGAGAA ACCCCATCTC TACTNAAAAT ACNAAATTAG  2280
   CCGGGCGTGG TAGCGCATGG CTGTAANCCT GGCTACTCAG GAGGCCGAGG CAGAANAATT  2340
   NCTTGAACTG GGGAGGCAGA GGTTGCGGTG AGCCCAGANC GCGCCATTGC ACTCCAGCCT  2400
   GGGTAACAAG AGCAAAACTC TGTCCAAAAA AAAAAAAAAA AA                     2442
(2) INFORMATION FOR SEQ ID NO:2:
   (i) SEQUENCE CHARACTERISTICS
      (A) LENGTH: 174 AMINO ACIDS
      (B) TYPE: AMINO ACID
      (C) STRANDEDNESS:
      (D) TOPOLOGY: LINEAR
   (ii) MOLECULE TYPE: PROTEIN
   (xi) SEQUENCE DESCRIPTION: SEQ ID NO:2:

## Description

This invention relates to newly identified polynucleotides, polypeptides encoded by such polynucleotides, the use of such polynucleotides and polypeptides, as well as the production of such polynucleotides and polypeptides. More particularly, the polypeptide of the present invention has been identified as a new member of the tumor necrosis factor family and is hereinafter referred to as "TNF-gamma". The invention also relates to inhibiting the action of such polypeptides.

Human tumor necrosis factors α (TNF-α) and β (TNF-β or lymphotoxin) are related members of a broad class of polypeptide mediators, which includes the interferons, interleukins and growth factors, collectively called cytokines (Beutler, B. and Cerami, A., Annu. Rev. Immunol., 7:625-655 (1989)).

Tumor necrosis factor (TNF-α and TNF-β) was originally discovered as a result of its anti-tumor activity, however, now it is recognized as a pleiotropic cytokine playing important roles in immune regulation and inflammation. To date, there are eight known members of the TNF-related cytokine family, TNF-α, TNF-β (lymphatoxin-α), LT-β, and ligands for the Fas, CD30, CD27, CD40 and 4-IBB receptors. These proteins have conserved C-terminal sequences and variable N-terminal sequences which are often used as membrane anchors, with the exception of TNF-β. Both TNF-α and TNF-β function as homotrimers when they bind to TNF receptors.

TNF is produced by a number of cell types, including monocytes, fibroblasts, T cells, natural killer (NK) cells and predominately by activated macrophages. TNF-α has been reported to have a role in the rapid necrosis of tumors, immunostimulation, autoimmune disease, graft rejection, resistance to parasites, producing an anti-viral response, septic shock, growth regulation, vascular endothelium effects and metabolic effects. TNF-α also triggers endothelial cells to secrete various factors, including PAI-1, IL-1, GM-CSF and IL-6 to promote cell proliferation. In addition, TNF-α up-regulates various cell adhesion molecules such as E-Selectin, ICAM-1 and VCAM-1. TNF-α and Fas ligand have also been shown to induce programmed cell death.

The first step in the induction of the various cellular responses mediated by TNF or LT is their binding to specific cell surface receptors. Two distinct TNF receptors of approximately 55-KDa (TNP-R1) and 75-KDa (TNF-R2) have been identified (Hohman, H.P. et al., J. Biol. Chem., 264:14927-14934 (1989)), and human and mouse cDNAs corresponding to both receptor types have been isolated and characterized (Loetscher, H. et al., Cell, 61:351 (1990)). Both TNF-Rs share the typical structure of cell surface receptors including extracellular, transmembrane and intracellular regions.

The polypeptide of the present invention has been identified as a novel member of the TNP family based on structural, amino acid sequence homology, and functional similarities, for example, TNF-gamma is a pro-inflammatory protein.

In accordance with one aspect of the present invention, there is provided a novel mature polypeptide which is TNF-gamma. Further described herein are biologically active and diagnostically or therapeutically useful fragments, analogs and derivatives thereof. The polypeptide of the present invention is of human origin.

In accordance with another aspect of the present invention, there are provided isolated nucleic acid molecules encoding human TNF-gamma, including mRNAs, DNAs, cDNAs, genomic DNAs. Further described herein are analogs and biologically active and diagnostically or therapeutically useful fragments and derivatives thereof.

In accordance with yet a further aspect of the present invention, there is provided a process for producing such polypeptide by recombinant techniques comprising culturing recombinant prokaryotic and/or eukaryotic host cells, containing a human TNF-gamma nucleic acid sequence, under conditions promoting expression of said protein and subsequent recovery of said protein.

In accordance with yet a further aspect of the present invention, there is provided a process for utilizing such polypeptide, or polynucleotide encoding such polypeptide, for screening for agonists and antagonists and for therapeutic purposes, for example, wound healing, to inhibit tumor proliferation, to provide resistance to parasites, bacteria and viruses, to induce inflammatory activities, to induce proliferation of endothelial cells and certain hematopoietic cells, to treat restenosis and to prevent certain autoimmune diseases.

In accordance with yet a further aspect of the present invention, there are also provided nucleic acid probes comprising nucleic acid molecules of sufficient length to specifically hybridize to human TNF-gamma sequences.

There are described TNF-gamma agonists which mimic TNF-gamma and binds to the TNF-gamma receptors to elicit TNF-gamma type responses.

In accordance with yet another aspect of the present invention, there are provided antagonists to such polypeptides, which may be used to inhibit the action of such polypeptides, for example, to prevent septic shock, inflammation, cerebral malaria, activation of the HIV virus, graft rejection, bone resorption and cachexia.

In accordance with still another aspect of the present invention, there are provided diagnostic assays for detecting diseases related to the under-expression and over-expression of the TNF-gamma polypeptide and nucleic acid sequences encoding such polypeptide.

These and other aspects of the present invention should be apparent to those skilled in the art from the teachings herein.

The following drawings are illustrative of embodiments of the invention and are not meant to limit the scope of the invention as encompassed by the claims.
Figure 1 illustrates the cDNA and corresponding deduced amino acid sequence of the polypeptide of the present invention. The initial 25 amino acids (underlined) are the putative leader sequence. The standard one-letter abbreviations for amino acids are used.
Figure 2 illustrates an amino acid sequence alignment between TNF-gamma and other members of the TNF family. TNF-gamma contains the conserved amino acid residues of the TNF family as shown by the shaded areas.
Figure 3A is an RNA blot analysis showing the human tissues where TNF-gamma is expressed. RNA from the tissues indicated were probed with labeled TNF-gamma cDNA. TNF-gamma mRNA exists predominantly in the kidney since Figure 3A shows a distinct band. Other lanes seem to show strong hybridization, however, these are actually non-specific smears.

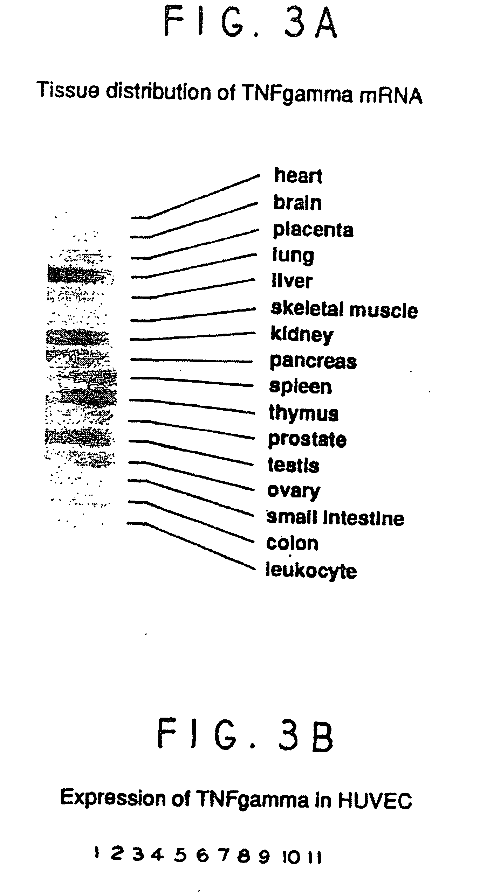

Figure 3B is an RNA blot analysis showing that TNF-gamma is expressed predominantly in HUVEC cells (human umbilical vein endothelial cells) which is lane 9. Lane 6 and lane 8 are non-specific smears. RNA from the cell lines indicated were probed with labeled TNF-gamma cDNA. Lane 1 is CAMA1 (breast cancer); lane 2 AN3CA (uterine cancer); lane 3, SK.UT.1 (uterine cancer); lane 4, MG63 (osteoblastoma) ; lane 5, HOS (osteoblastoma); lane 6, MCF7 (breast cancer) ; lane 7, OVCAR-3 (ovarian cancer); lane 8, CAOV-3 (ovarian cancer); lane 9, HUVEC; lane 10, AOSMIC (smooth muscle); lane 11, foreskin fibroblast.
Figure 4 is a photograph of a gel after electrophoresing TNF-gamma was produced by bacterial expression and purification.
Figure 5 is a photograph of a gel after baculovirus expression of TNF-gamma.
Figure 6A is a photograph of WEHI 164 cells which are untreated (upper left) and after exposure to TNF-α, TNF-β and TNF-gamma. Cells which have an elongated non-round morphology have been lysed. The TNF added was approximately 0.5 µg/ml. Photographs were taken 72 hours after addition of TNF.
Figure 6B illustrates the ability of TNF-gamma in comparison to TNF-α and TNF-β to inhibit WEHI 164 cell growth.
Figure 7 illustrates the ability of recombinant TNF-gamma, TNF-α and TNF-β to induce WEHI 164 cell death.
Figure 8 illustrates the ability of recombinant TNF-α, TNF-β, and TNF-gamma to induce morphological change in L929 cells. The morphology change is indicated by dark round cells. Cells were treated with *E*. *Coli* produced recombinant TNF at approximately 0.5 µg/ml. The photographs were taken 72 hours after the addition of TNF. The morphology change indicates that the cells have been killed.
Figure 9 is a graphical illustration of the effect of TNF-gamma, TNF-α and TNF-β on venous endothelial cells. Cell proliferation after venous endothelial cells were treated with commercially available TNF-α and TNF-β and *E. Coli* produced TNF-gactuna was quantified using an MTS assay.
Figure 10 is a photograph of HL60 cells, with control showing the HL60 cells being spread apart; TNF-α and TNF-gamma induce cell adhesion and cell-cell contact as illustrated by the cells adhering together in the lower right.
Figure 11 illustrates that TNF-gamma does not significantly bind to two known soluble TNF receptors, namely sTNF RI (p55) and sTNF RII (p75).

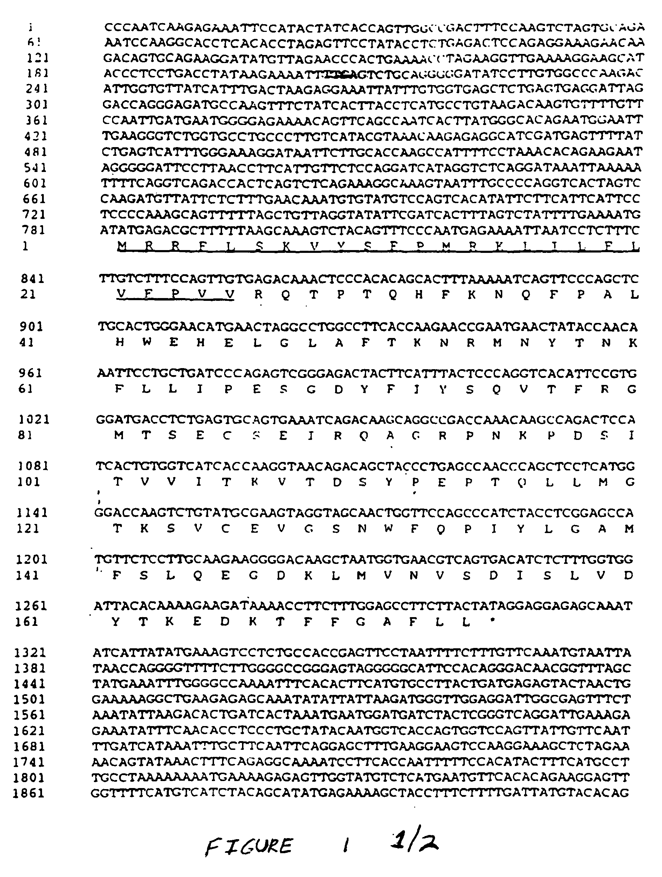

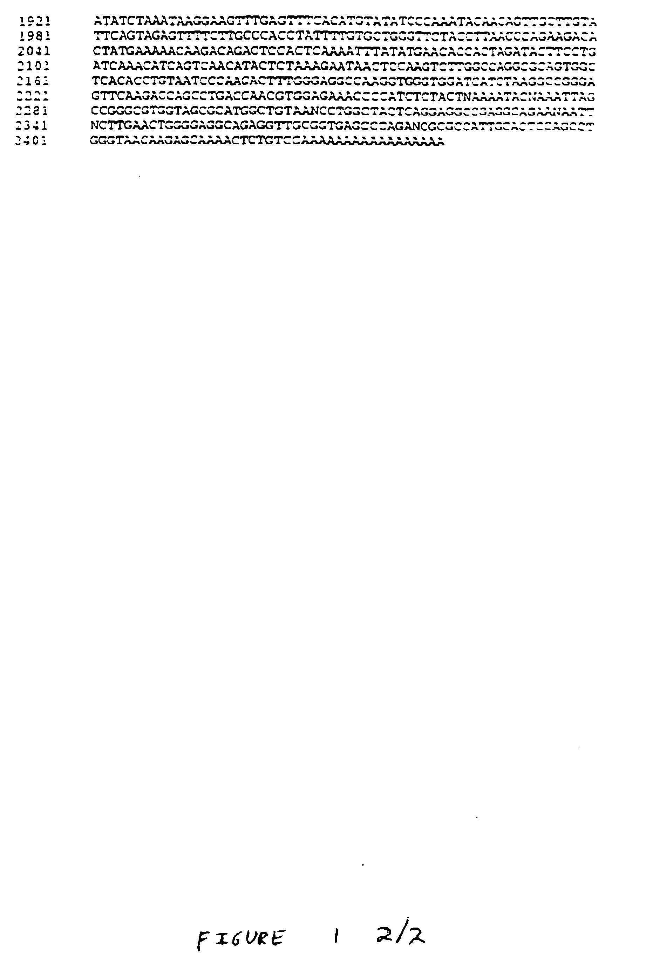

In accordance with an aspect of the present invention, there is provided an isolated nucleic acid (polynucleotide) which encodes for the mature polypeptide having the deduced amino acid sequence of Figure 1 or for the mature polypeptide encoded by the cDNA of the clone deposited as ATCC Deposit No. 75927 on October 26, 1994.

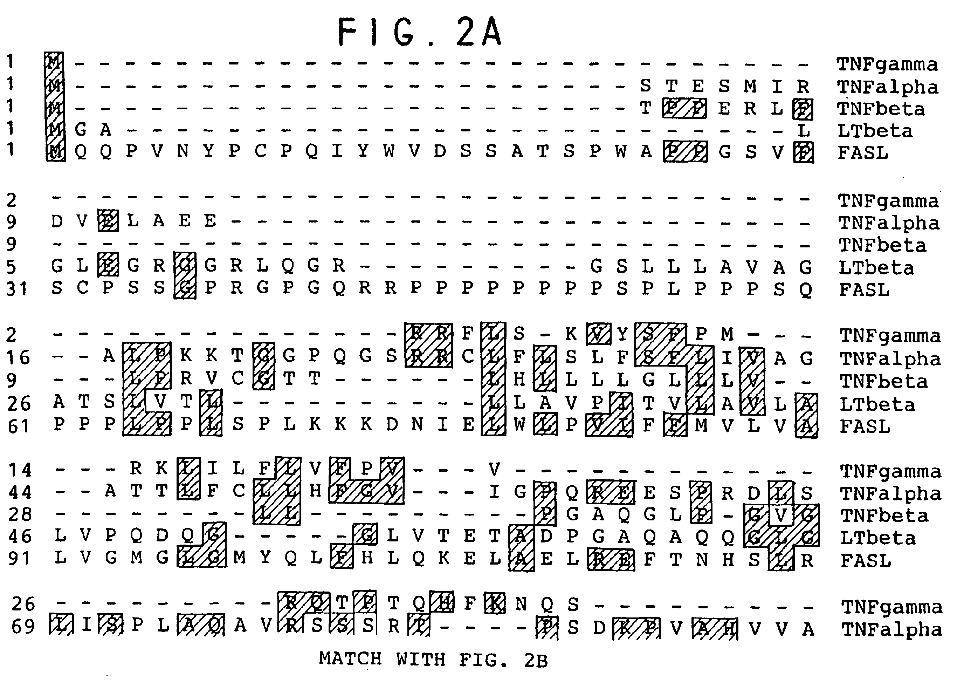

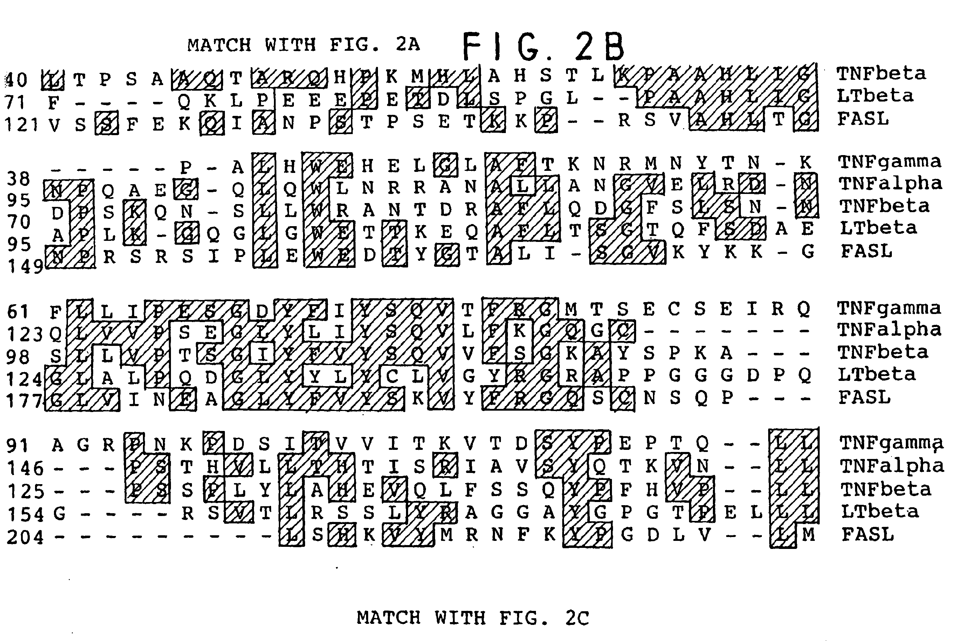

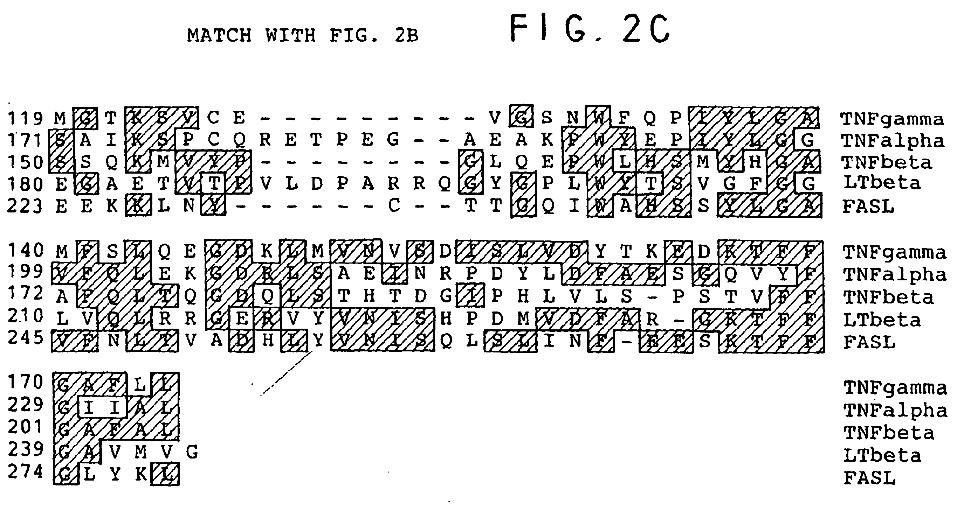

A polynucleotide encoding a polypeptide of the present invention may be obtained from human kidney and umbilical vein endothelial cells. The polynucleotide of this invention was discovered in a cDNA library derived from a human umbilical vein endothelial cell. It is structurally related to the TNF family. It contains an open reading frame encoding a protein of 174 amino acid residues of which approximately the first 25 amino acids residues are the putative leader sequence such that the mature protein comprises 149 amino acids. The protein exhibits the highest degree of homology at the C-terminus to Rabbit TNF-α with 38 % identity and 58 % similarity over a 111 amino acid stretch. Sequences conserved throughout the members of the TNF family are also conserved in TNF-gamma (see Figure 2). The bolded letters indicate conserved amino acid residues. The TNF-gamma mRNA is specifically expressed in human umbilical vein endothelial cells as shown in the RNA blot analysis of Figure 3B.

The polynucleotide of the present invention may be in the form of RNA or in the form of DNA, which DNA includes cDNA, genomic DNA, and synthetic DNA. The DNA may be double-stranded or single-stranded, and if single stranded may be the coding strand or non-coding (anti-sense) strand. The coding sequence which encodes the mature polypeptide may be identical to the coding sequence shown in Figure 1 or that of the deposited clone or may be a different coding sequence which coding sequence, as a result of the redundancy or degeneracy of the genetic code, encodes the same mature polypeptide as the DNA of Figure 1 or the deposited cDNA.

The polynucleotide which encodes for the mature polypeptide of Figure 1 or for the mature polypeptide encoded by the deposited cDNA may include, but is not limited to: only the coding sequence for the mature polypeptide; the coding sequence for the mature polypeptide and additional coding sequence such as a leader or secretory sequence or a proprotein sequence; the coding sequence for the mature polypeptide (and optionally additional coding sequence) and non-coding sequence, such as introns or non-coding sequence 5' and/or 3' of the coding sequence for the mature polypeptide.

Thus, the term "polynucleotide encoding a polypeptide" encompasses a polynucleotide which includes only coding sequence for the polypeptide as well as a polynucleotide which includes additional coding and/or non-coding sequence.

Further described herein are variants of the hereinabove described polynucleotides which encode for fragments, analogs and derivatives of the polypeptide having the deduced amino acid sequence of Figure 1 or the polypeptide encoded by the cDNA of the deposited clone. The variant of the -polynucleotide may be a naturally occurring allelic variant of the polynucleotide or a non-naturally occurring variant of the polynucleotide.

Thus, the present invention includes polynucleotides encoding the same mature polypeptide as shown in Figure 1 or the same mature polypeptide encoded by the cDNA of the deposited clone. Further described herein are variants of such polynucleotides which variants encode for a fragment, derivative or analog of the polypeptide of Figure 1 or the polypeptide encoded by the cDNA of the deposited clone. Such nucleotide variants include deletion variants, substitution variants and addition or insertion variants.

As hereinabove indicated, the polynucleotide may have a coding sequence which is a naturally occurring allelic variant of the coding sequence shown in Figure 1 or of the coding sequence of the deposited clone. As known in the art, an allelic variant is an alternate form of a polynucleotide sequence which may have a substitution, deletion or addition of one or more nucleotides, which does not substantially alter the function of the encoded polypeptide.

The present invention also includes polynucleotides, wherein the coding sequence for the mature polypeptide may be fused in the same reading frame to a polynucleotide sequence which aids in expression and secretion of a polypeptide from a host cell, for example, a leader sequence which functions as a secretory sequence for controlling transport of a polypeptide from the cell. The polypeptide having a leader sequence is a preprotein and may have the leader sequence cleaved by the host cell to form the mature form of the polypeptide. The polynucleotides may also encode for a proprotein which is the mature protein plus additional 5' amino acid residues. A mature protein having a prosequence is a proprotein and is an inactive form of the protein. Once the prosequence is cleaved an active mature protein remains.

Thus, for example, the polynucleotide of the present invention may encode for a mature protein, or for a protein having a prosequence or for a protein having both a prosequence and a presequence (leader sequence).

The polynucleotides of the present invention may also have the coding sequence fused in frame to a marker sequence which allows for purification of the polypeptide of the present invention. The marker sequence may be a hexahistidine tag supplied by a pQE-9 vector to provide for purification of the mature polypeptide fused to the marker in the case of a bacterial host, or, for example, the marker sequence may be a hemagglutinin (HA) tag when a mammalian host, e.g. COS-7 cells, is used. The HA tag corresponds to an epitope derived from the influenza hemagglutinin protein (Wilson, I., et al., Cell, 37:767 (1984)).

The present invention further relates to polynucleotides which hybridize to the hereinabove-described sequences if there is at least 50% and preferably 70% identity between the sequences. The present invention particularly relates to polynucleotides which hybridize under stringent conditions to the hereinabove-described polynucleotides. As herein used, the term "stringent conditions" means hybridization will occur only if there is at least 95% and preferably at least 97% identity between the sequences. The polynucleotides which hybridize to the hereinabove described polynucleotides in a preferred embodiment encode polypeptides which retain substantially the same biological function or activity as the mature polypeptide encoded by the cDNA of Figure 1 or the deposited cDNA.

The deposit(s) referred to herein will be maintained under the terms of the Budapest Treaty on the International Recognition of the Deposit of Micro-organisms for purposes of Patent Procedure. These deposits are provided merely as convenience to those of skill in the art and are not an admission that a deposit is required under 35 U.S.C. §112. The sequence of the polynucleotides contained in the deposited materials, as well as the amino acid sequence of the polypeptides encoded thereby, are incorporated herein by reference and are controlling in the event of any conflict with any description of sequences herein. A license may be required to make, use or sell the deposited materials, and no such license is hereby granted.

The present invention further relates to a TNF-gamma polypeptide which has the deduced amino acid sequence of Figure 1 or which has the amino acid sequence encoded by the deposited cDNA. Further described herein are fragments, analogs and derivatives of such polypeptide.

The terms "fragment," "derivative" and "analog" when referring to the polypeptide of Figure 1 or that encoded by the deposited cDNA, means a polypeptide which retains essentially the same biological function or activity as such polypeptide. Thus, an analog includes a proprotein which can be activated by cleavage of the proprotein portion to produce an active mature polypeptide.

The polypeptide of the present invention may be a recombinant polypeptide, a natural polypeptide or a synthetic polypeptide, preferably a recombinant polypeptide.

The fragment, derivative or analog of the polypeptide of Figure 1 or that encoded by the deposited cDNA may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethylene glycol), or (iv) one in which the additional amino acids are fused to the mature polypeptide, such as a leader or secretory sequence or a sequence which is employed for purification of the mature polypeptide or a proprotein sequence. Such fragments, derivatives and analogs are deemed to be within the scope of those skilled in the art from the teachings herein.

The polypeptides and polynucleotides of the present invention are preferably provided in an isolated form, and preferably are purified to homogeneity.

The term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The present invention also relates to vectors which include polynucleotides of the present invention, host cells which are genetically engineered with vectors of the invention and the production of polypeptides of the invention by recombinant techniques.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the TNF-gamma genes. The culture conditions, such as temperature, pH and the like, are those previously used with the host cell selected for expression, and will be apparent to the ordinarily skilled artisan.

The polynucleotides of the present invention may be employed for producing polypeptides by recombinant techniques. Thus, for example, the polynucleotide may be included in any one of a variety of expression vectors for expressing a polypeptide. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda P_{L} promoter and other promoters known to control expression of genes in prokaryotic or eukaryotic cells or their viruses. The expression vector also contains.a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in E. coli.

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as E. coli, Streptomyces, Salmonella typhimurium; fungal cells, such as yeast; insect cells such as Drosophila S2 and Sf9; animal cells such as CHO, COS or Bowes melanoma; adenoviruses; plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example. Bacterial: pQE70, pQE60, pQE-9 (Qiagen), pBS, pD10, phagescript, psiX174, pbluescript SK, pbsks, pNH8A, pNH16a, pNH18A, pNH46A (Stratagene); ptrc99a, pKK223-3, pKK233-3, pDR540, pRIT5 (Pharmacia). Eukaryotic: pWLNEO, pSV2CAT, pOG44, pXT1, pSG (Stratagene) pSVK3, pBPV, pMSG, pSVL (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are PKK232-8 and PCM7. Particular named bacterial promoters include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation. (Davis, L., Dibner, M., Battey, I., Basic Methods in Molecular Biology, (1986)).

The constructs in host cells can be used in a conventional manner to produce the gene product encoded-by the recombinant sequence. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989).

Transcription of the DNA encoding the polypeptides of the present invention by higher eukaryotes is increased by inserting an enhancer sequence into the vector. Enhancers are cis-acting elements of DNA, usually about from 10 to 300 bp that act on a promoter to increase its transcription. Examples including the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, e.g., stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E. coli, Bacillus subtilis, Salmonella typhimurium and various species within the genera Pseudomonas, Streptomyces, and Staphylococcus, although others may also be employed as a matter of choice.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well know to those skilled in the art.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The TNF-gamma polypeptides can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography hydroxylapatite chromatography and lectin chromatography. Protein refolding steps can be used, as necessary, in completing configuration of the mature protein. Finally, high performance liquid chromatography (HPLC) can be employed for final purification steps.

The polypeptides of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated or may be non-glycosylated. Polypeptides of the invention may also include an initial methionine amino acid residue.

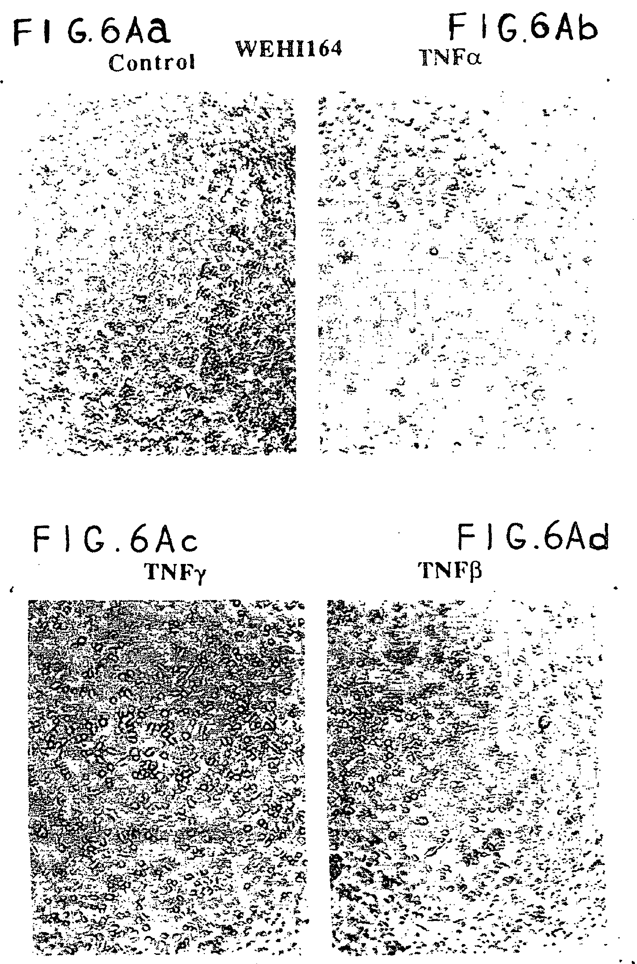

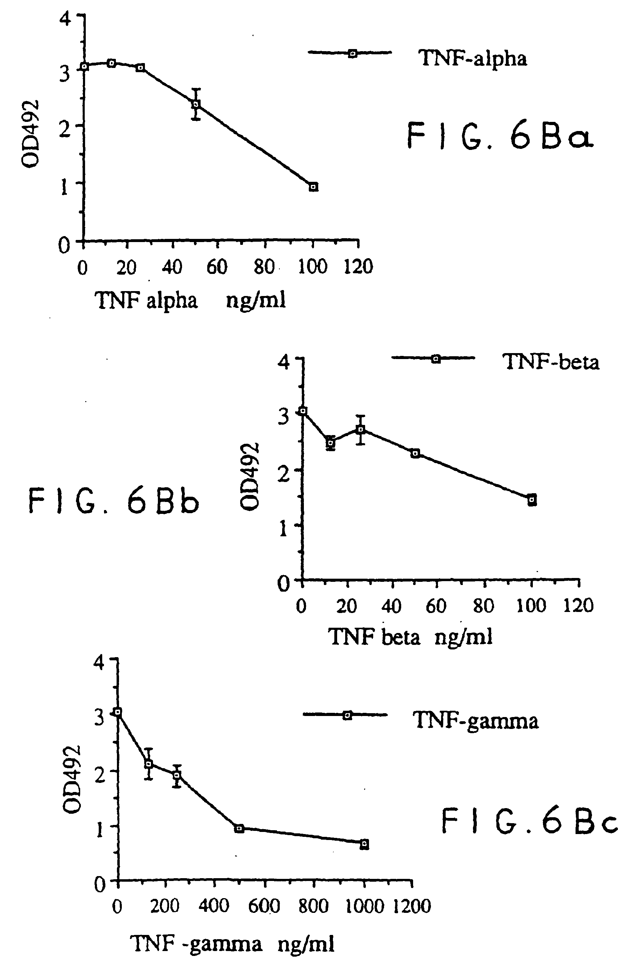

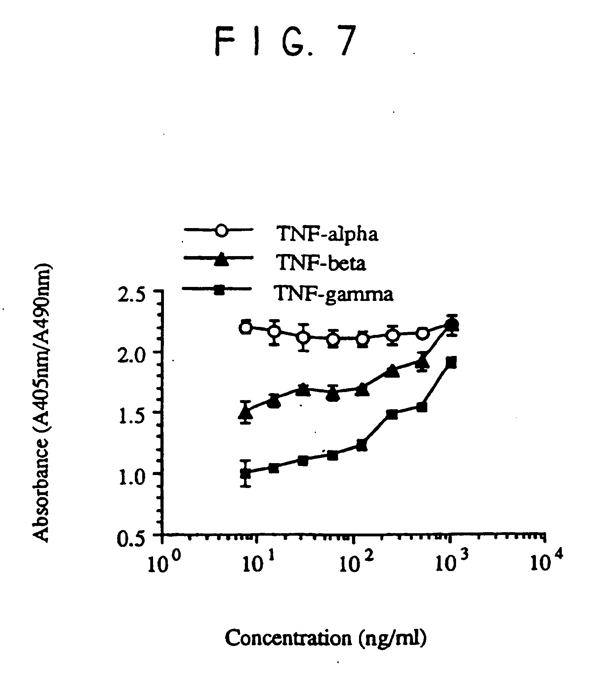

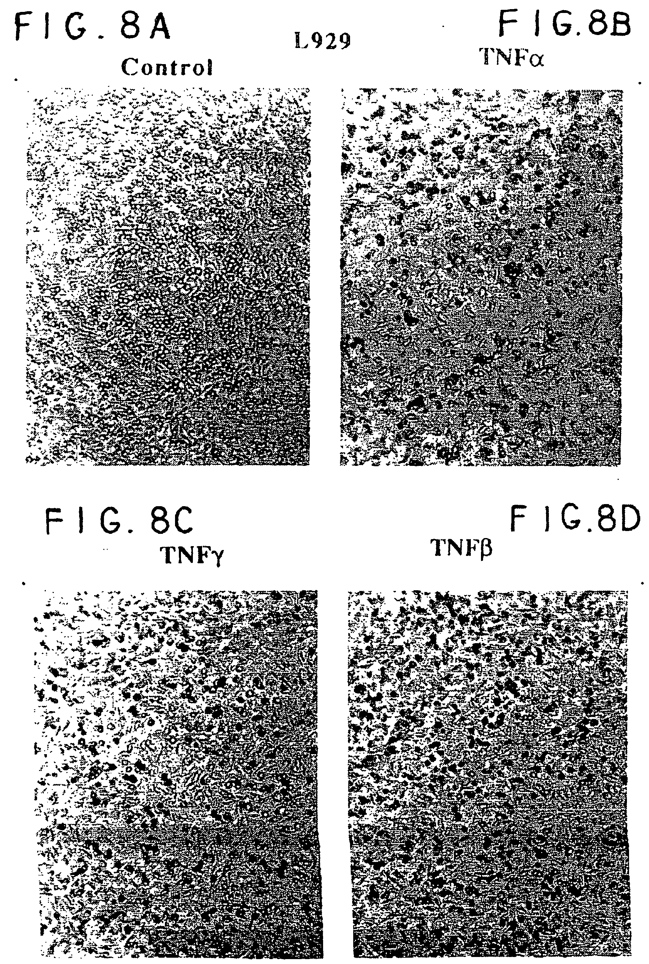

The TNF-gamma polypeptide of the present invention may be employed to inhibit tumor cell growth or neoplasia. The TNF-gamma polypeptide may be responsible for tumor destruction throuph apoptosis which is characterized by membrane blebbin (zeiosis), condensation of cytoplasma and the activation of an endogeneous endonuclease (Figure 7). As shown in Table 1, TNF-gamma has strong cytotoxic activity for the cell lines tested which have abnormal cellular proliferation and regulation, for example the fibrosarcoma and carcinoma cell line. This is also illustrated in Figure 6A, 6B and 8 where it is shown that TNF-gamma has the ability to inhibit L929 and WEHI 164 cell growth through cytotoxic activity. WEHI 164 cells are mouse fibrosarcoma cells. A preferable method of administering the TNF-gamma is by injection directly into the tumor.

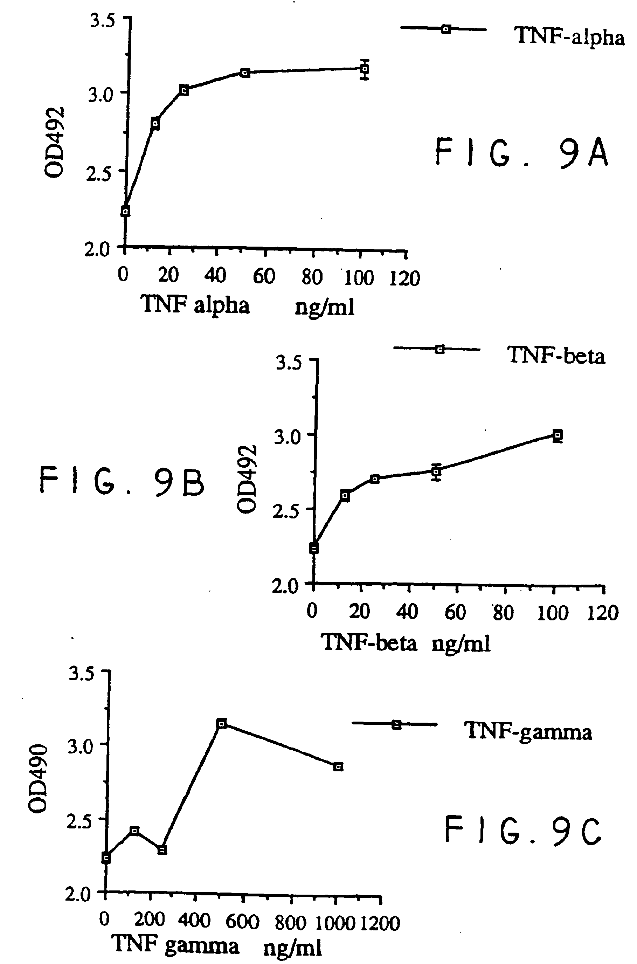

The cell adhesion activity of TNF-gamma may be employed for wound healing. As shown in Table 1 and Figure 9, TNF-gamma has a strong endothelial cell proliferation effect which is an indication that TNF-gamma plays a role in wound healing. TNF-gamma's cell adhesive effects may also play a role in wound healing.

TNF-gamma may also be employed to treat diseases which require growth promotion activity, for example, restenosis. As stated above, TNF-gamma is shown to have strong proliferation effects on endothelial cell growth. Accordingly, TNF-gamma may also be employed to regulate hematopoiesis and endothelial cell development.

The TNF-gactnna polypeptide, through its ability to stimulate the activation of T-cells, is an important mediator of the immune response. Accordingly, this polypeptide may be used to stimulate an immune response against a variety of parasitic, bacterial and viral infections. TNF-gamma may lyse virus-infected cells and, therefore, be employed to arrest HIV infected cells.

The TNF-gamma polypeptide may also be employed to treat autoimmune diseases such as Type I diabetes by enhancing the T-cell proliferative response.

**Table 1**

| Summary of TNF-gamma activity | | | | | |
|---|---|---|---|---|---|
| Cell lines | Source and Type | Activity | | | |
| | | Cytotoxicity | Proliferation | Differentiation | Adhesion |
| L929 | mouse fibroblast | + | - | - | - |
| WEHI 164 | mouse fibrosarcoma | +++ | - | - | - |
| NRK-54E | rat kidney epithelial-like | + | - | - | - |
| THP-1 | human monocytic leukemia | + | - | ++ | ++ |
| HL-60 | human promyelocytic leukemia | - | - | - | ++ |
| Raji | human Burkitt lymphoma | - | - | - | - |
| Jurkat | human T cell leukemia | ++ | - | - | - |
| Primary | human venous endothelial cells | - | ++ | - | ? |
| Primary | human arterial endothelial cells | +* | ++ | - | ? |
| A-431 | human epidermoid carcinoma | ++ | - | - | - |
| 293 | human embryonal kidney | - | ++ | - | - |

| | | | | | |
|---|---|---|---|---|---|
| * At high dose only. The numbers of + indicate the relative level of activities. - indicates no detected activity at the concentration range tested. | | | | | |

The polynucleotides and polypeptides of the present invention may be employed as research reagents and materials for discovery of treatments and diagnostics to human disease.

This invention provides a method for identification of the receptor for TNF-gamma. The gene encoding the receptor can be identified by numerous methods known to those of skill in the art, for example, ligand panning and FACS sorting (Coligan, et al., Current Protocols in Immun., 1(2), Chapter 5, (1991)). Preferably, expression cloning is employed wherein polyadenylated RNA is prepared from a cell responsive to TNF-gamma, and a cDNA library created from this RNA is divided into pools and used to transfect COS cells or other cells that are not responsive to TNF-gamma. Transfected cells which are grown on glass slides are exposed to labeled TNF-gamma. TNF-gamma can be labeled by a variety of means including iodination or inclusion of a recognition site for a site-specific protein kinase. Following fixation and incubation, the slides are subjected to autoradiographic analysis. Positive pools are identified and sub-pools are prepared and retransfected using an iterative sub-pooling and rescreening process, eventually yielding a single clone that encodes the putative receptor.

As an alternative approach for receptor identification, labeled TNF-gamma can be photoaffinity linked with cell membrane or extract preparations that express the receptor molecule. Cross-linked material is resolved by PAGE and exposed to X-ray film. The labeled complex containing the TNF-gamma-receptor can be excised, resolved into peptide fragments, and subjected to protein microsequencing. The amino acid sequence obtained from microsequencing would be used to design a set of degenerate oligonucleotide probes to screen a cDNA library to identify the gene encoding the putative receptor.

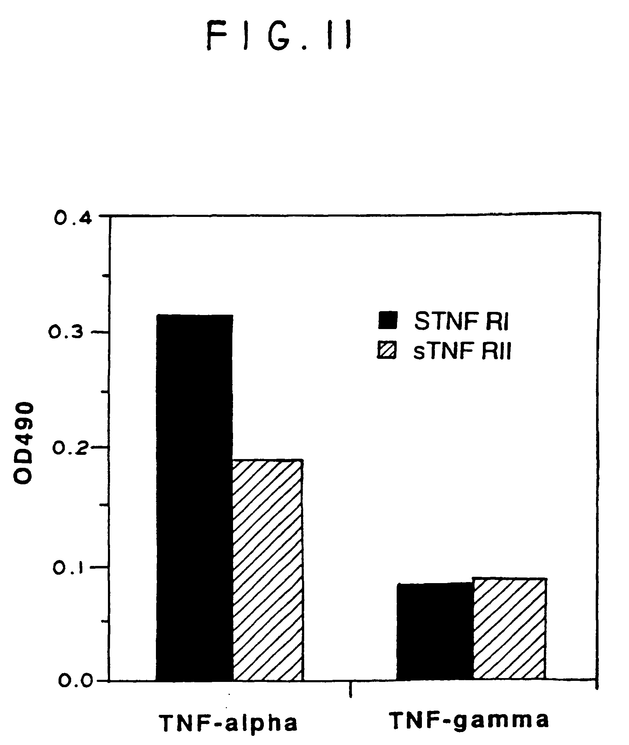

TNF-gamma does not bind significantly to two soluble TNF receptors, sTNF-RI (p55) and sTNF-RII (p75). Accordingly, TNF-gamma may have activities inclusive of and additional to known TNF proteins (see Figure 11).

Further disclosed herein is a method of screening compounds to identify those which mimic TNF-gamma (agonists) or prevent the effect of TNF-gamma. An example of such a method takes advantage of the ability of TNF-gamma to significantly stimulate the proliferation of human endothelial cells in the presence of the comitogen Con A. Endothelial cells are obtained and cultured in 96-well flat-bottomed culture plates (Costar, Cambridge, MA) in RPM1 1640 supplemented with 10% heat-inactivated fetal bovine serum (Hyclone Labs, Logan, UT), 1% L-glutamine, 100 U/ml penicillin, 100 µg/ml steptomycin, 0.1% gentamycin (Gibco Life Technologies, Grand Island, NY) in the presence of 2 µg/ml of Con-A (Calbiochem, La Jolla, CA). Con-A, and the compound to be screened are added to a final volume of 0.2 ml. After 60 h at 37°C, cultures are pulsed with 1 µCi of [³H]thymidine (5 Ci/mmol; 1 Ci = 37 BGq; NEN) for 12-18 h and harvested onto glass fiber filters (PhD; Cambridge Technology, Watertown, MA). Mean [³H]thymidine incorporation (cpm) of triplicate cultures is determined using a liquid scintillation counter (Beckman Instruments, Irvine, CA). Significant [³H] thymidine incorporation indicates stimulation of endothelial cell proliferation.

Alternatively, the response of a known second messenger system following interaction of TNF-gamma and receptor would be measured and compared in the presence or absence of the compound. Such second messenger systems include but are not limited to, cAMP guanylate cyclase, ion channels or phosphoinositide hydrolysis.

To assay for antagonists, the assay described above is performed, however, in this assay TNF-gamma is added along with the compound to be screened and the ability of the compound to inhibit [³H]thymidine incorporation in the presence of TNF-ganuna, indicates that the compund is an antagonist to TNF-gamma. Alternatively, TNF-gamma antagonists may be detected by combining TNF-gamma and a potential antagonist with membrane-bound TNF-gamma receptors or recombinant receptors under appropriate conditions for a competitive inhibition assay. TNF-gamma can be labeled, such as by radioactivity, such that the number of TNF-gamma molecules bound to the receptor can determine the effectiveness of the potential antagonist.

Alternatively, a mammalian cell or membrane preparation expressing the TNF-gamma receptor is incubated with labeled TNF-gamma in the presence of the compound. The ability of the compound to enhance or block this interaction could then be measured.

Antibodies specific to TNF-gamma may be used as antagonists by binding to TNF-gamma and preventing it from binding to its receptor. Monoclonal antibodies are particularly effective in this regard. Antibodies specific to the TNF-gamma receptor, however, may mediate distinct cellular responses which tend to agonize the effects of TNF-gamma upon interaction with its receptor.

Potential TNF-gamma antagonists also include TNF-gamma mutants which bind to the TNF-gamma receptor and elicit no second messenger response to effectively block the receptor from its natural ligand. Specifically designed oligonucleotides and small molecules may also bind to the TNF-gamma receptor and block it from TNF-gamma. Examples of small molecules include but are not limited to small peptides or peptide-like molecules.

Another potential TNF-gamma antagonist is a soluble form of the TNF-gamma receptor which binds to TNF-gamma and prevents it from interacting with membrane-bound TNF-gamma receptors. In this way, the receptors are not stimulated by TNF-gamma.

Another potential TNF-gamma antagonist is an antisense construct prepared using antisense technology. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes for the mature polypeptides of the present invention, is used to design an antisense RNA oligonucleotide of from about 10 to 40 base pairs in length. A DNA oligonucleotide is designed to be complementary to a region of the gene involved in transcription (triple helix -see Lee et al., Nucl. Acids Res., 6:3073 (1979); Cooney et al, Science, 241:456 (1988); and Dervan et al., Science, 251: 1360 (1991)), thereby preventing transcription and the production of TNF-gamma. The antisense RNA oligonucleotide hybridizes to the mRNA *in vivo* and blocks translation of the mRNA molecule into the TNF-gamma polypeptide (Antisense - Okano, J. Neurochem., 56:560 (1991); Oligodeoxynucleotides as Antisense Inhibitors of Gene Expression, CRC Press, Boca Raton, FL (1988)). The oligonucleotides described above can also be delivered to cells such that the antisense RNA or DNA may be expressed *in vivo* to inhibit production of TNF-gamma.

TNF-antagonists may also be employed to treat cachexia which is a lipid clearing defect resulting from a systemic deficiency of lipoprotein lipase which is suppressed by TNF-gamma. The TNF-gamma antagonists are also employed to treat cerebral malaria in which TNF-gamma appears to play a pathogenic role. The antagonists may also be employed to treat rheumatoid arthritis by inhibiting TNF-gamma induced production of inflammatory cytokines such as IL-1 in the synovial cells. When treating arthritis TNF-gamma is preferably injected intra-articularly.

The TNF-gamma antagonists may also be employed to prevent graft rejection by preventing the stimulation of the immune system in the presence of a graft by TNF-gamma.

The TNF-gamma antagonists may also be employed to treat osteoporosis since TNF-gamma may induce bone resorption.

Antagonists to TNF-gamma may also be employed as anti-inflammation agents since TNF-gamma mediates an enhanced inflammatory response.

The antagonists may also be used to treat endotoxic shock, also referred to as septic shock. This critical condition results from an exaggerated response to bacterial or other types of infection. This response leads to elevated levels of TNF-gamma which causes shock and tissue injury.

The antagonists may be employed in a composition with a pharmaceutically acceptable carrier, e.g., as hereinafter described.

Fragments of the full length TNF-gamma gene may be used as a hybridization probe for a cDNA library to isolate the full length gene and to isolate other genes which have a high sequence similarity to the gene or similar biological activity. Probes of this type can be, far example, between 20 and 2000 bases. Preferably, however, the probes have between 30 and 50 base pairs. The probe may also be used to identify a cDNA clone corresponding to a full length transcript and a genomic clone or clones that contain the complete TNF-gamma gene including regulatory and promotor regions, exons, and introns. As an example of a screen comprises isolating the coding region of the TNF-gamma gene by using the known DNA sequence to synthesize an oligonucleotide probe. Labeled oligonucleotides having a sequence complementary to that of the gene of the present invention are used to screen a library of human cDNA, genomic, DNA or mRNA to determine which members of the library the probe hybridizes to.

The TNF-gamma polypeptides and antagonists of the present invention and agonists described herein may be employed in combination with a suitable pharmaceutical carrier. Such compositions comprise a therapeutically effective amount of the compound, and a pharmaceutically acceptable carrier or excipient. Such a carrier includes but is not limited to saline, buffered saline, dextrose, water, glycerol, ethanol, and combinations thereof. The formulation should suit the mode of administration.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the pharmaceutical compositions of the present invention may be employed in conjunction with other therapeutic compounds.

The pharmaceutical compositions may be administered in a convenient manner such as by the topical, intravenous, intraperitoneal, intramuscular, subcutaneous, intranasal or intradermal routes. The pharmaceutical compositions are administered in an amount which is effective for treating and/or prophylaxis of the specific indication. In general, they are administered in an amount of at least about 10 µg/kg body weight and in most cases they will be administered in an amount not in excess of about 8 mg/Kg body weight per day. In most cases, the dosage is from about 10 µg/kg to about 1 mg/kg body weight daily, taking into account the routes of administration, symptoms, etc.

The TNF-gamma polypeptides and agonists and antagonists which are polypeptides may also be employed in accordance with the present invention by expression of such polypeptides *in vivo,* which is often referred to as "gene therapy."

Thus, for example, cells from a patient may be engineered with a polynucleotide (DNA or RNA) encoding a polypeptide *ex vivo*, with the engineered cells then being provided to a patient to be treated with the polypeptide. Such methods are well-known in the art and are apparent from the teachings herein. For example, cells may be engineered by the use of a retroviral particle containing RNA encoding a polypeptide of the present invention.

Similarly, cells may be engineered *in vivo* for expression of a polypeptide *in vivo* by, for example, procedures known in the art. For example, a producer cell for producing a retroviral particle containing RNA encoding a polypeptide of the present invention may be administered to a patient for engineering cells *in vivo* and expression of the polypeptide *in vivo.* These and other methods for administering a polypeptide of the present invention by such method should be apparent to those skilled in the art from the teachings of the present invention. For example, the expression vehicle for engineering cells may be other than a retrovirus, for example, an adenovirus which may be used to engineer cells *in vivo* after combination with a suitable delivery vehicle.

This invention is also related to the use of the TNF-gamma gene as part of a diagnostic assay for detecting diseases or susceptibility to diseases related to the presence of mutated TNF-gamma. Such diseases are related to an under-expression of TNF-gamma, for example, abnormal cellular proliferation such as tumors and cancers.

Individuals carrying mutations in the human TNF-gamma gene may be detected at the DNA level by a variety of techniques. Nucleic acids for diagnosis may be obtained from a patient's cells, such as from blood, urine, saliva, tissue biopsy and autopsy material. The genomic DNA may be used directly for detection or may be amplified enzymatically by using PCR (Saiki et al., Nature, 324:163-166 (1986)) prior to analysis. RNA or cDNA may also be used for the same purpose. As an example, PCR primers complementary to the nucleic acid encoding TNF-gamma can be used to identify and analyze TNF-gamma mutations. For example, deletions and insertions can be detected by a change in size of the amplified product in comparison to the normal genotype. Point mutations can be identified by hybridizing amplified DNA to radiolabeled TNF-gamma RNA or alternatively, radiolabeled TNF-gamma antisense DNA sequences. Perfectly matched sequences can be distinguished from mismatched duplexes by RNase A digestion or by differences in melting temperatures.

Genetic testing based on DNA sequence differences may be achieved by detection of alteration in electrophoretic mobility of DNA fragments in gels with or without denaturing agents. Small sequence deletions and insertions can be visualized by high resolution gel electrophoresis. DNA fragments of different sequences may be distinguished on denaturing formamidine gradient gels in which the mobilities of different DNA fragments are retarded in the gel at different positions according to their specific melting or partial melting temperatures (see, e.g., Myers et al., Science, 230:1242 (1985)).

Sequence changes at specific locations may also be revealed by nuclease protection assays, such as RNase and S1 protection or the chemical cleavage method (e.g., Cotton et al., PNAS, USA, 85:4397-4401 (1985)).

Thus, the detection of a specific DNA sequence may be achieved by methods such as hybridization, RNase protection, chemical cleavage, direct DNA sequencing or the use of restriction enzymes, (e.g., Restriction Fragment Length Polymorphisms (RFLP)) and Southern blotting of genomic DNA.

In addition to more conventional gel-electrophoresis and DNA sequencing, mutations can also be detected by *in situ* analysis.

The present invention also relates to a diagnostic in vitro assay for detecting altered levels of TNF-gamma protein in various tissues since an over-expression of the proteins compared to normal control tissue samples may detect the presence of a disease or susceptibility to a disease, for example, tumors and cerebral malaria. Assays used to detect levels of TNF-gamma protein in a sample derived from a host are well-known to those of skill in the art and include radioimmunoassays, competitive-binding assays, Western Blot analysis, ELISA assays and "sandwich" assay. An ELISA assay (Coligan, et al., Current Protocols in Immunology, 1(2), Chapter 6, (1991)) initially comprises preparing an antibody specific to the TNF-gamma antigen, preferably a monoclonal antibody. In addition a reporter antibody is prepared against the monoclonal antibody. To the reporter antibody is attached a detectable reagent such as radioactivity, flourescence or in this example a horseradish peroxidase enzyme. A sample is now removed from a host and incubated on a solid support, e.g. a polystyrene dish, that binds the proteins in the sample. Any free protein binding sites on the dish are then covered by incubating with a non-specific protein like BSA. Next, the monoclonal antibody is incubated in the dish during which time the monoclonal antibodies attach to any TNF-gamma proteins attached to the polystyrene dish. All unbound monoclonal antibody is washed out with buffer. The reporter antibody linked to horseradish perosxidase is now placed in the dish resulting in binding of the reporter antibody to any monoclonal antibody bound to TNF-gamma. Unattached reporter antibody is then washed out. Peroxidase substrates are then added to the dish and the amount of color developed in a given time preiod is a measurement of the amount of TNF-gamma protein present in a given volume of patient sample when compared against a standard curve.

A competition assay may be employed wherein antibodies specific to TNF-gamma are attached to a solid support and labeled TNF-gamma and a sample derived from the host are passed over the solid support and the amount of label detected, for example by liquid scintillation chromotagraphy, can be correlated to a quantity of TNF-gamma in the sample.

A "sandwich" assay is similar to an ELISA assay. In a "sandwich" assay TNF-gamma is passed over a solid support and binds to antibody attached to a solid support. A second antibody is then bound to the TNF-gamma. A third antibody which is labeled and specific to the second antibody is then passed over the solid support and binds to the second antibody and an amount can then be quantitated.

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

Briefly, sequences can be mapped to chromosomes by preparing PCR primers (preferably 15-25 bp) from the cDNA. Computer analysis of the 3' untranslated region of the sequence is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include *in situ* hybridization, prescreening with labeled flow-sorted chromosomes and preselection by hybridization to construct chromosome specific-cDNA libraries.

Fluorescence *in situ* hybridization (FISH) of a cDNA clone to a metaphase chromosomal spread can be used to provide a precise chromosomal location in one step. This technique can be used with cDNA as short as 500 or 600 bases; however, clones larger than 2,000 bp have a higher likelihood of binding to a unique chromosomal location with sufficient signal intensity for simple detection. FISH requires use of the clones from which the express sequence tag (EST) was derived, and the longer the better. For example, 2,000 bp is good, 4,000 is better, and more than 4,000 is probably not necessary to get good results a reasonable percentage of the time. For a review of this technique, see Verma et al., Human Chromosomes: a Manual of Basic Techniques, Pergamon Press, New York (1988).

Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb).

The polypeptides, their fragments or other derivatives, or analogs thereof, or cells expressing them can be used as an immunogen to produce antibodies thereto. These antibodies can be, for example, polyclonal or monoclonal antibodies. The present invention also includes chimeric, single chain, and humanized antibodies, as well as Fab fragments, or the product of an Fab expression library. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies can then be used to isolate the polypeptide from tissue expressing that polypeptide.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic polypeptide products of this invention. Also, transgenic mice may be used to express humanized antibodies to immunogenic polypeptide products of this invention.

The present invention will be further described with reference to the following examples; however, it is to be understood that the present invention is not limited to such examples. All parts or amounts, unless otherwise specified, are by weight.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel, D. et al., Nucleic Acids Res., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., et al., Id., p. 146). Unless otherwise provided, ligation may be accomplished using known buffers and conditions with 10 units of T4 DNA ligase ("ligase") per 0.5 µg of approximately equimolar amounts of the DNA fragments to be ligated.

Unless otherwise stated, transformation was performed as described in the method of Graham, F. and Van der Eb, A., Virology, 52:456-457 (1973).

### Example 1

### Bacterial Expression and Purification of TNF-Ganuna

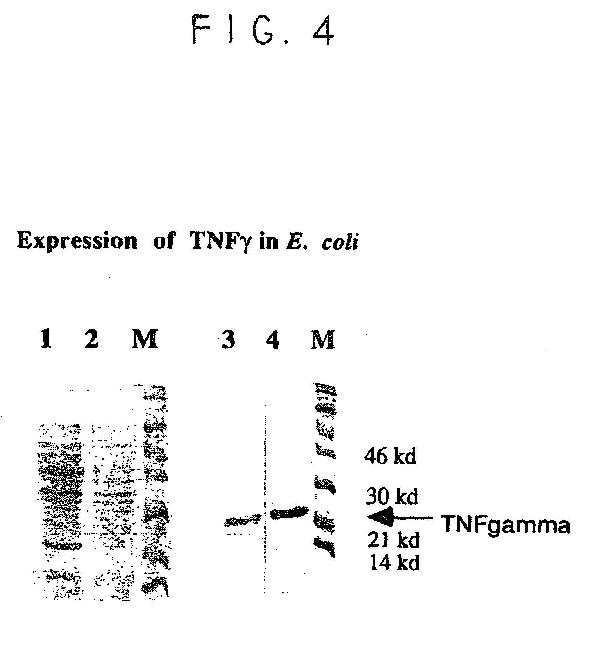

The DNA sequence encoding TNF-gamma, ATCC # 75927, is initially amplified using PCR oligonucleotide primers corresponding to the 5' sequences of the TNF-gamma protein and the vector sequences 3' to the TNF-gamma gene. Additional nucleotides corresponding to TNF-gamma were added to the 5' and 3' sequences respectively. The 5' oligonucleotide primer has the sequence 5' GCGCGGATCCACCATGAGACGCTTTTTAAGCAAAGTC 3' contains a Bam HI restriction enzyme site followed by the first 24 nucleotides of TNF-gamma coding sequence starting from the presumed terminal amino acid of the processed protein codon. The 3' sequence 5' CGCGTCTAGACTATAGTAAGAAGGCTCCAAAGAAGG 3' contains complementary sequences to an XbaI site and is followed by 22 nucleotides of TNF-gamma and to a pQE-9 vector sequence located 3' to the TNF-gamma DNA insert. The restriction enzyme sites correspond to the restriction enzyme sites on the bacterial expression vector pQE-9 (Qiagen). pQE-9 was then digested with Bam HI and Xba I. The amplified sequences were ligated into pQE-9 and were inserted in frame with the sequence encoding for the histidine tag and the RBS. The ligation mixture was then used to transform an E. coli strain available from Qiagen under the trademark M15/rep 4 by the procedure described in Sambrook, J. et al., Molecular Cloning: A Laboratory Manual, Cold Spring Laboratory Press, (1989). M15/rep4 contains multiple copies of the plasmid pRBP4, which expresses the lacI repressor and also confers kanamycin resistance (Kan^{r}). Transformants are identified by their ability to grow on LB plates and ampicillin/kanamycin resistant colonies were selected. Plasmid DNA was isolated and confirmed by restriction analysis. Clones containing the desired constructs were grown overnight (O/N) in liquid culture in LB media supplemented with both Amp (100 ug/ml) and Kan (25 ug/ml). The O/N culture is used to inoculate a large culture at a ratio of 1:100 to 1:250. The cells were grown to an optical density 600 (O.D.⁶⁰⁰) of between 0.4 and 0.6. IPTG ("Isopropyl-B-D-thiogalacto pyranoside") was then added to a final concentration of 1 mM. IPTG induces by inactivating the lacI repressor, clearing the P/O leading to increased gene expression. Cells were grown an extra 3 to 4 hours. Cells were then harvested by centrifugation. The cell pellet was solubilized in the chaotropic agent 6 Molar Guanidine HCl. After clarification, solubilized TNF-gamma was purified from this solution by chromatography on a Nickel-Chelate column under conditions that allow for tight binding by proteins containing the 6-His tag (Hochuli, E. et al., J. Chromatography 411:177-184 (1984)). TNF-gamma was further purified by a second run on the Nickel-chelate column. TNF-gamma (90% pure) was eluted from the column in 6 molar guanidine HCl pH 5.0 and for the purpose of renaturation was dialyzed in PBS buffer. The expression product was electrophoresed by SDS-PAGE, and the results may be seen in Figure 4 where M is molecular weight markers; lane 1 is induced cell lysate; lane 2 is uninduced call lysate; lane 3 is the TNF-gamma protein after two Nickel-chelate column purifications; lane 4 is the TNF-gamma protein after 1 column purification.

### Example 2

### Cloning and expression of TNF-gamma using the baculovirus expression system

The DNA sequence encoding the full length TNF-gamma protein, ATCC # 75927, was amplified using PCR oligonucleotide primers corresponding to the 5' and 3' sequences of the gene:

The 5' primer has the sequence 5' GCGCGGATCCACCATGAGACGCTTTTTAAGCAAAGTC 3' and contains a Bam HI restriction enzyme site (in bold) followed by 24 nucleotides of the TNF-gamma gene (the initiation codon for translation "ATG" is underlined).

The 3' primer has the sequence 5' CGCGTCTAGACTATAGTAAGAAGGCTCCAAAGAAGG 3' and contains the cleavage site for the restriction endonuclease XbaI and 22 nucleotides complementary to the 3' non-translated sequence of the TNF-gamma gene. The amplified sequences were isolated from a 1% agarose gel using a commercially available kit ("Geneclean," BIO 101 Inc., La Jolla, Ca.). The fragment was then digested with the endonucleases BamHI and XbaI and then purified again on a 1% agarose gel. This fragment is designated F2.

The vector pA2 (modification of pVL941 vector, discussed below) is used for the expression of the TNF-gamma protein using the baculovirus expression system (for review see: Summers, M.D. and Smith, G.E. 1987, A manual of methods for baculovirus vectors and insect cell culture procedures, Texas Agricultural Experimental Station Bulletin No. 1555). This expression vector contains the strong polyhedrin promoter of the Autographa californica nuclear polyhedrosis virus (AcMNPV) followed by the recognition sites for the restriction endonucleases BamHI and XbaI. The polyadenylation site of the simian virus (SV)40 is used for efficient polyadenylation. For an easy selection of recombinant virus the beta-galactosidase gene from E.coli is inserted in the same orientation as the polyhedrin promoter followed by the polyadenylation signal of the polyhedrin gene. The polyhedrin sequences are flanked at both sides by viral sequences for the cell-mediated homologous recombination of cotransfected wild-type viral DNA. Many other baculovirus vectors could be used in place of pA2, such as pRG1, pAc373, pVL941 and pAcIMl (Luckow, V.A. and Summers, M.D., Virology, 170:31-39).

The plasmid was digested with the restriction enzymes BamHI and XbaI and then dephosphorylated using calf intestinal phosphatase by procedures known in the art. The DNA was then isolated from a 1% agarose gel using the commercially available kit ("Geneclean" BIO 101 Inc., La Jolla, Ca.). This vector DNA is designated V2.

Fragment F2 and the dephosphorylated plasmid V2 were ligated with T4 DNA ligase. E.coli XL1 blue cells were then transformed. The sequence of the cloned fragment was confirmed by DNA sequencing.

5 µg of the plasmid pBac TNF-ganuna was cotransfected with 1.0 µg of a commercially available linearized baculovirus ("BaculoGold^{™} baculovirus DNA", Pharmingen, San Diego, CA.) using the lipofection method (Felgner et al. Proc. Natl. Acad. Sci. USA, 84:7413-7417 (1987)).

1µg of BaculoGold^{™} virus DNA and 5 µg of the plasmid pBac TNF-gamma were mixed in a sterile well of a microtiter plate containing 50 µl of serum free Grace's medium (Life Technologies Inc., Gaithersburg, MD). Afterwards 10 µl Lipofectin plus 90 µl Grace's medium were added, mixed and incubated for 15 minutes at room temperature. Then the transfection mixture was added dropwise to the Sf9 insect cells (ATCC CRL 1711) seeded in a 35 mm tissue culture plate with 1m1 Grace' medium without serum. The plate was rocked back and forth to mix the newly added solution. The plate was then incubated for 5 hours at 27°C. After 5 hours the transfection solution was removed from the plate and 1 ml of Grace's insect medium supplemented with 10% fetal calf serum was added. The plate was put back into an incubator and cultivation continued at 27°C for four days.

After four days the supernatant was collected and a plaque assay performed similar as described by Summers and Smith (supra). As a modification an agarose gel with "Blue Gal" (Life Technologies Inc., Gaithersburg) was used which allows an easy isolation of blue stained plaques. (A detailed description of a "plaque assay" can also be found in the user's guide for insect cell culture and baculovirology distributed by Life Technologies Inc., Gaithersburg, page 9-10).

Four days after the serial dilution, the virus was added to the cells, blue stained plaques were picked with the tip of an Eppendorf pipette. The agar containing the recombinant viruses was then resuspended in an Eppendorf tube containing 200 µl of Grace's medium. The agar was removed by a brief centrifugation and the supernatant containing the recombinant baculovirus was used to infect Sf9 cells seeded in 35 mm dishes. Four days later the supernatants of these culture dishes were harvested and then stored at 4°C.

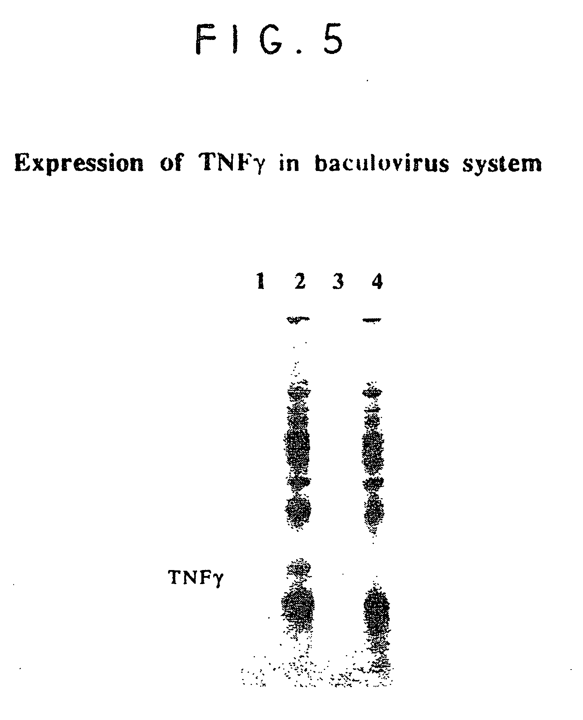

Sf9 cells were grown in Grace's medium supplemented with 10% heat-inactivated FBS. The cells were infected with the recombinant baculovirus V-TNF-gamma at a multiplicity of infection (MOI) of 2. Six hours later the medium was removed and replaced with SF900 II medium minus methionine and cysteine (Life Technologies Inc., Gaithersburg). 42 hours later 5 µCi of ³⁵S-methionine and 5 µCi ³⁵S cysteine (Amersham) were added. The cells were further incubated for 16 hours before they were harvested by centrifugation and the labelled proteins visualized by SDS-PAGE and autoradiography. Figure 5 illustrates the gel where Lanes 1 and 3 are the medium of the TNF-gamma and control; lanes 2 and 4 are the cell lysate of the TNF-gamma and the control.

### Example 3

### Expression of Recombinant TNF-gamma in COS cells

The expression of plasmid, TNF-gamma HA is derived from a vector pcDNAI/Amp (Invitrogen) containing: 1) SV40 origin of replication, 2) ampicillin resistance gene, 3) E.coli replication origin, 4) CMV promoter followed by a polylinker region, a SV40 intron and polyadenylation site. A DNA fragment encoding the entire TNF-gamma precursor and a HA tag fused in frame to its 3' end was cloned into the polylinker region of the vector, therefore, the recombinant protein expression is directed under the CMV promoter. The HA tag correspond to an epitope derived from the influenza hemagglutinin protein as previously described (I. Wilson, H. Niman, R. Heighten, A Cherenson, M. Connolly, and R. Lerner, 1984, Cell 37, 767). The infusion of HA tag to our target protein allows easy detection of the recombinant protein with an antibody that recognizes the HA epitope.

The plasmid construction strategy is described as follows:

The DNA sequence encoding TNF-gamma, ATCC # 75927, was constructed by PCR on the original EST cloned using two primers: the 5' primer (same as for the bacula example) contains a BamHI site followed by 24 nucleotides of TNF-gamma coding sequence starting from the initiation codon; the 3' sequence 5' CGCTCTAGATCAAGCGTAGTCTGGGACGTCGTATGGATAGTAAGAAG GCTCCAAAG 3' contains complementary sequences to XbaI site, translation stop codon, HA tag and the last 18 nucleotides of the TNF-gamma coding sequence (not including the stop codon) . Therefore, the PCR product contains a BamHI site, TNF-gamma coding sequence followed by HA tag fused in frame, a translation termination stop codon next to the HA tag, and an XbaI site. The PCR amplified DNA fragment and the vector, pcDNAI/Amp, were digested with BamHI and XbaI restriction enzyme and ligated. The ligation mixture was transformed into E. coli strain SURE (available from Stratagene Cloning Systems, 11099 North Torrey Pines Road, La Jolla, CA 92037) the transformed culture was plated on ampicillin media plates and resistant colonies were selected. Plasmid DNA was isolated from transformants and examined by restriction analysis for the presence of the correct fragment. For expression of the recombinant TNF-gamma, COS cells were transfected with the expression vector by DEAE-DEXTRAN method. (J. Sambrook, E. Fritsch, T. Maniatis, Molecular Cloning: A Laboratory Manual, Cold Spring Laboratory Press, (1989)). The expression of the TNF-gamma HA protein was detected by radiolabelling and immunoprecipitation method. (E. Harlow, D. Lane, Antibodies: A Laboratory Manual, Cold Spring Harbor Laboratory Press, (1988)). Cells were labelled for 8 hours with ³⁵S-cysteine two days post transfection. Culture media were then collected and cells were lysed with detergent (RIPA buffer (150 mM NaCl, 1% NP-40, 0.1% SDS, 1% NP-40, 0.5% DOC, 50mM Tris, pH 7.5). (Wilson, I. et al., Id. 37:767 (1984)). Both cell lysate and culture media were precipitated with a HA specific monoclonal antibody. Proteins precipitated were analyzed on 15% SDS-PAGE gels.

### Example 4

### Expression pattern of TNF-gamma in human tissue

RNA blot analysis was carried out to examine the levels of expression of TNF-gamma in human tissues. Total cellular RNA samples were isolated with RNAzol^{™} B system (Biotecx Laboratories, Inc. 6023 South Loop East, Houston, TX 77033). About 2 µg (for the RNA blot of Figure 3A) of total RNA isolated from each human tissue specified was separated on 1% agarose-formaldehyde gel and blotted onto a nylon filter. (Sambrook, Fritsch, and Maniatis, Molecular Cloning, Cold Spring Harbor Press, (1989)). The labeling reaction was done according to the Stratagene Prime-It kit with 50 ng TNF-gamma cDNA, to produce ³²P-labeled TNF-gamma cDNA. The labeled DNA was purified with a Select-G-50 column (5 Prime - 3 Prime, Inc. 5603 Arapahoe Road, Boulder, CO 80303). The filter was then hybridized with radioactive labeled full-length TNF-gamma gene at 1,000,000 cpm/ml in 0.5 M NaPO₄, pH 7.4 and 7% SDS overnight at 65°C. After being washed twice at room temperature and twice at 60°C with 0.5 x SSC, 0.1% SDS, the filter was then exposed at -70°C overnight with an intensifying screen. The message RNA for TNF-gamma is abundant in kidney (Figures 3A).

The same reaction was done for the results shown in Figure 3B, with the only change being that 10 µg poly A RNA from the tissues indicated were used. The message RNA for TNF-gamma is expressed predominantly in HUVEC cells (Figure 3B).

### Example 5

### Ability of Recombinant TNF-gamma to inhibit WEHI 164 and L929 cell growth, induce cell adhesion in HL-60 cells and promote endothelial cell growth.

The adherent target cells were prepared from confluent cultures by trypsinization in PBS, and non-adherent target cells were harvested from stationary cultures and washed once with medium. Target cells were suspended at 3 x 10⁵ cells/ml in medium containing 10% FCS. 0.1 ml aliquots were dispensed into 96-well flat-bottomed microtiter plates containing 0.1 ml serially diluted test samples of cells (WEHI 164 and L929). Incubation was continued for 70 hours. TNF-α, TNF-β and TNF-gamma were added at a 0.5 µg/ml concentration. The cytotoxicity and proliferation activity was quantified using an MTS assay performed by the addition of 20 µl of MTS and phenazine methosulfate (PMS) solution to each well. After a three hour incubation, the OD at 492 nm was measured by an ELISA plate reader. The OD₄₉₂ is proportional to the number of viable cells in the wells. The percent of cytotoxicity was calculated as follows: % cytotoxicity = (100 - OCₑₓₚₑᵣᵢₘₑₙₜₐₗ/OD_{control}) X 100. The photographs were taken after 72 hours. As shown by Figure 6A, and 8 TNF-gamma induced a morphology change which appeared as dark round cells which are killed.

In the graph of Figure 6B, the assay was performed as described above, however, increasing amounts of TNF were added. The results indicate that TNF-gamma is an inhbitor of WEHI 164 cells.

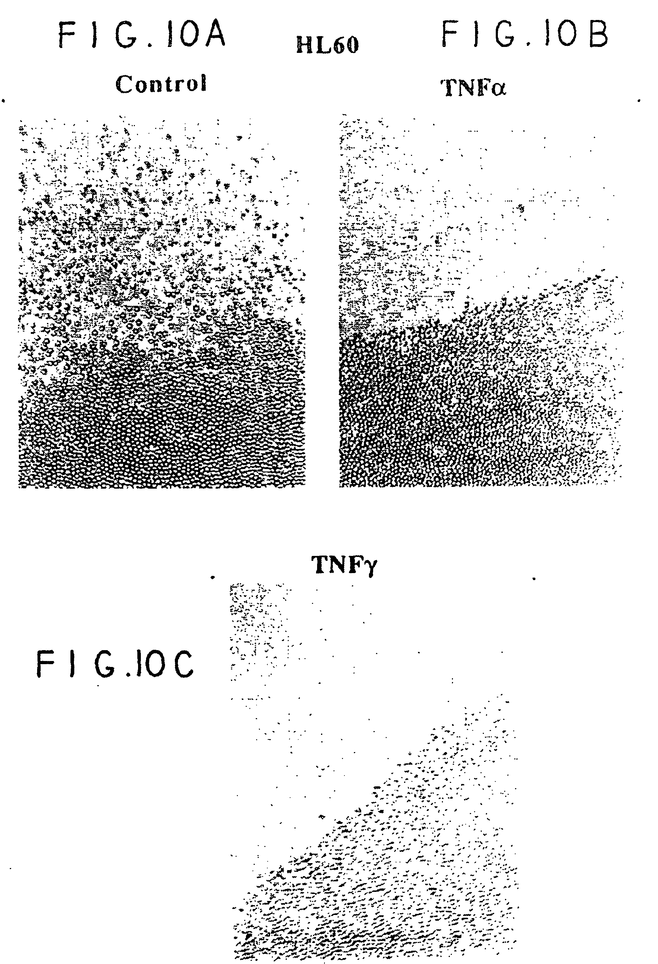

To test adhesion ability of TNF-gamma, HL-60 cells were used and cell adhesion and cell-cell contact were measured by observation under the microscope and scored subjectively by two independent investigators. Figure 10 illustrates TNF-gamma's ability for inducing cell adhesion.

In the assay to test for ability of TNF-gamma to promote endothelial cell growth, the proliferation index (PI) was calculated as follows: PI = ODₑₓₚₑᵣᵢₘₑₙₜₐₗ/OD_{control}. Figure 8 illustrates that TNF-gamma is a promotor of endothelial cell growth.

### Example 6

### Measurement of Apoptosis ability of TNF-aamsna

In a first incubation step, anti-histone antibody is fixed adsorptively on the wall of a microtiter plate module. Subsequently, non-specific binding sites on the wall are saturated by treatment with incubation buffer (eg., blocking solution). During the second incubation step, the nucleosomes contained in the WEHI 164 cell sample treated with the TNF-α, TNF-β or TNF-gamma bind via their histone components to the immobilized anti-histone antibody. In the third incubation step, anti-DNA-peroxidase (POD) reacts with the DNA-part of the nucleosome. After removal of all unbound peroxidase conjugate by a washing step, the amount of peroxidase retained in the immunocomplex is determined photometrically with ABTS (2,2'-azino-di-[3-ethylbenzthiazoline sulfonate]), as a substrate. Anti-histone antibody reacts with the histones H1, H2A, H2B, H3 and H4 from the sample. Anti-DNA POD antibody binds to single- and double-stranded DNA. Therefore, the ELISA allows the detection of mono- and oligonucleosomes and may be applied to measure apoptotic cell death. The level of cell death is measured by the amountof cytoplasmic histone-associated DNA fragments which are indicated as the absorbance A405nm/A490. (See Boehringer mannheim Catalogue, 0990 C 93 2 1541170) (see Figure 7)

### Example 7

### Receptor binding assay using TNF-gamma

TNF-α and TNF-gamma were Ni-NTA affinity chromatography purified using the 6-His tag and 1 µg/well was added to a nickel chelate coated 96-well plate (Xenopore Corp.), and incubated for 2 hours. After washing three times, 100 ng of human soluble TNF receptors, sTNF RI or sTNF RII, was added to each well and incubated for 2 hours. The plate was washed three times and alkaline phosphatase-labeled polyclonal antibody to sTNF RI or sTNF RII (200 µl) was added. Substrate solution, 200 µl was added to each well and the plate was incubated for 2 hours. The OD was measured using an ELISA reader (test wavelength 450 nm, correction wavelength 590 nm). The results shown in Figure 11 illustrate that TNF-gamma does not bind significantly to sTNF-receptors.

Numerous modifications and variations of the present invention are possible in light of the above teachings and, therefore, within the scope of the appended claims, the invention may be practiced otherwise than as particularly described.

## Claims

1. A polynucleotide selected from the group consisting of
(a) polynucleotides encoding at least the mature form of the polypeptide having the deduced amino acid sequence as shown in SEQ ID NO: 2;
(b) polynucleotides having the coding sequence as shown in SEQ ID NO: 2 encoding at least the mature form of the polypeptide;
(c) polynucleotides encoding the polypeptide having the amino acid sequence of at least the mature form of the polypeptide encoded by the cDNA contained in ATCC 75927;
(d) polynucleotides having the coding sequence of the cDNA contained in ATCC 75927 encoding at least the mature form of the polypeptide; and
(e) polynucleotides which are at least 95% identical to a polynucleotide of any one of (a) to (d), which encode a polypeptide that stimulates proinflammatory cytokine release from T-cells.

2. The polynucleotide of claim 1 which is DNA.

3. The DNA of claim 2 which is genomic DNA.

4. The polynucleotide of claim 1 which is RNA.

5. A vector containing the polynucleotide of any one of claims 1 to 4.

6. The vector of claim 5 in which the polynucleotide is operatively linked to expression control sequences allowing expression in prokaryotic or eukaryotic host cells.

7. A host cell genetically engineered with the vector of claim 5 or 6.

8. A process for producing a polypeptide having TNF-gamma activity comprising culturing the host cell of claim 8 and recovering the polypeptide encoded by said polynucleotide from the culture.

9. A process for producing cells capable of expressing a polypeptide having TNF-gamma activity comprising genetically engineering cells with the vector of claim 5 or 6.

10. A polypeptide having the amino acid sequence encoded by a polynucleotide of any one of claims 1 to 4 or obtainable by the process of claim 8.

11. An antibody against the polypeptide of claim 10.

12. An antagonist/inhibitor against the polypeptide of claim 10 which is an antibody of claim 11, or an antisense construct which is able to hybridize to a transcript of the polynucleotide of any one of claims 1 to 4.

13. A nucleic acid molecule which specifically hybridizes to a polynucleotide of any one of claims 1 to 4.

14. A pharmaceutical composition comprising the polynucleotide of any one of claims 1 to 4 or the polypeptide of claim 10 and optionally a pharmaceutically acceptable carrier.

15. A diagnostic composition comprising the polynucleotide of any one of claims 1 to 4, the nucleic acid molecule of claim 13 or the antibody of claim 11.

16. Use of the polypeptide of claim 10 or of the polynucleotide of any one of claims 1 to 4 for the preparation of a pharmaceutical composition for wound healing, for inhibiting tumor proliferation or neoplasia, for the treatment of restenosis or for the prevention of autoimmune diseases.

17. Use of the antagonist of claim 12 for the preparation of a pharmaceutical composition for the treatment of cachexia, cerebral malaria, rheumatoid arthritis, osteoporosis, endotoxic (septic) shock, for the prevention of graft rejection or useful as anti-inflammation agent.

18. A method for identifying agonists and antagonists to the polypeptide of claim 10 comprising:
(a) combining endothelial cells, Concanavalin-A, the compound to be screened, [3H] thymidine selectively in the presence or absence of the polypeptide of claim 10;
(b) measuring the [3H] thymidine incorporation by the endothelial cells; and
(c) determining if the compound enhanced or blocked [3H] thymidine incorporation.

19. An in vitro method for diagnosing a tumor or a susceptibility to a tumor comprising detecting a mutated form of the nucleic acid sequence encoding the polypeptide of claim 10.

20. The antibody of claim 11 which is a polyclonal antibody.

21. The antibody of claim 11 which is selected from the group consisting of:
(a) a monoclonal antibody;
(b) a chimeric antibody;
(c) a single chain antibody; and
(d) a Fab fragment.

22. A cell that produces the antibody of claim 11.

23. A hybridoma that produces the antibody of claim 11.

## Patentansprüche

1. Polynucleotid, ausgewählt aus der Gruppe bestehend aus:
(a) Polynucleotiden, die zumindest die reife Form des Polypeptids mit der in SEQ ID NO: 2 gezeigten abgeleiteten Aminosäuresequenz codieren;
(b) Polynucleotiden mit der codierenden Sequenz wie in SEQ ID NO: 2 gezeigt, die mindestens die reife Form des Polypeptids codiert;
(c) Polynucleotiden, die das Polypeptid mit der Aminosäuresequenz von zumindest der reifen Form des Polypeptids codieren, das durch die in ATCC 75927 enthaltene cDNA codiert wird;
(d) Polynucleotiden mit der codierenden Sequenz der cDNA, die in ATCC 75927 enthalten ist, die zumindest die reife Form des Polypeptids codiert; und
(e) Polynucleotiden, die zumindest 95 % identisch sind mit einem der Polynucleotide nach (a) bis (d), die ein Polypeptid codieren, das vorentzündliche Cytokin-Freisetzung aus T-Zellen stimuliert.

2. Polynucleotid nach Anspruch 1, das DNA ist.

3. DNA nach Anspruch 2, die genomische DNA ist.

4. Polynucleotid nach Anspruch 1, das RNA ist.

5. Vektor, der das Polynucleotid nach einem der Ansprüche 1 bis 4 enthält.

6. Vektor nach Anspruch 5, in dem das Polynucleotid funktionell mit einer Expressionskontrollsequenz verknüpft ist, die die Expression in prokaryontischen oder eukaryontischen Wirtszellen ermöglicht.

7. Wirtszelle, die mit dem Vektor nach Anspruch 5 oder 6 gentechnisch verändert ist.

8. Verfahren zur Herstellung eines Polypeptids mit TNF-gamma-Aktivität, umfassend das Züchten der Wirtszelle nach Anspruch 8 und das Gewinnen des Polypeptids, das durch das Polynucleotid codiert wird, aus der Kultur.

9. Verfahren zur Herstellung von Zellen, die in der Lage sind, ein Polypeptid mit TNF-gamma-Aktivität zu exprimieren, welches das gentechnische Verändern der Zellen mit dem Vektor nach Anspruch 5 oder 6 umfasst.

10. Polypeptid mit der Aminosäuresequenz, die durch ein Polynucleotid gemäß einem der Ansprüche 1 bis 4 codiert wird, oder durch das Verfahren nach Anspruch 8 erhältlich ist.

11. Antikörper gegen das Polypeptid nach Anspruch 10.

12. Antagonist/Inhibitor gegen das Polypeptid nach Anspruch 10, der ein Antikörper nach Anspruch 11 oder ein Antisense-Konstrukt ist, das in der Lage ist, an ein Transkript des Polynucleotids nach einem der Ansprüche 1 bis 4 zu hybridisieren.

13. Nucleinsäuremolekül, das spezifisch an ein Polynucleotid nach einem der Ansprüche 1 bis 4 hybridisiert.

14. Arzneimittel, welches das Polynucleotid nach einem der Ansprüche 1 bis 4 oder das Polypeptid nach Anspruch 10 und gegebenenfalls einen pharmazeutisch verträglichen Träger umfasst.

15. Diagnostische Zusammensetzung, die das Polynucleotid nach einem der Ansprüche 1 bis 4, das Nucleinsäuremolekül nach Anspruch 13 oder den Antikörper nach Anspruch 11 umfasst.

16. Verwendung des Polypeptids nach Anspruch 10 oder des Polynucleotids nach einem der Ansprüche 1 bis 4 für die Herstellung eines Arzneimittels zur Wundheilung, zur Hemmung von Tumorproliferation oder Neoplasie, zur Behandlung von Restenose oder zur Prävention von Autoimmunerkrankungen.

17. Verwendung des Antagonisten nach Anspruch 12 für die Herstellung eines Arzneimittels zur Behandlung von Kachexie, cerebraler Malaria, rheumatoider Arthritis, Osteoporose, endotoxischem (septischem) Schock, zur Prävention von Transplantatabstoßung oder das als entzündungshemmendes Mittel nützlich ist.

18. Verfahren zur Identifizierung von Agonisten und Antagonisten zu dem Polypeptid nach Anspruch 10, umfassend:
(a) selektives Kombinieren von Endothelzellen, Concanavalin-A, der Verbindung, die gescreent werden soll, [3H]-Thymidin, in Anwesenheit oder Abwesenheit des Polypeptids nach Anspruch 10;
(b) Messen des [3H]-Thymidin-Einbaus durch die Endothelzellen; und
(c) Bestimmen, ob die Verbindung den [3H]-Thymidin-Einbau verstärkt oder blockiert.

19. In vitro-Verfahren zur Diagnose eines Tumors oder der Anfälligkeit für einen Tumor, umfassend den Nachweis einer mutierten Form der Nucleinsäuresequenz, die das Polypeptid nach Anspruch 10 codiert.

20. Antiköper nach Anspruch 11, der ein polyclonaler Antikörper ist.

21. Antikörper nach Anspruch 11, der ausgewählt ist aus der Gruppe bestehend aus:
(a) einem monoclonalen Antikörper;
(b) einem chimären Antikörper,
(c) einem Einzelkettenantikörper; und
(d) einem Fab-Fragment.

22. Zelle, die den Antikörper nach Anspruch 11 produziert.

23. Hybridom, das den Antikörper nach Anspruch 11 produziert.

## Revendications

1. Polynucléotide choisi dans le groupe constitué par :
(a) des polynucléotides codant pour au moins la forme mature du polypeptide ayant la séquence d'aminoacides telle que décrite dans SEQ ID n° 2 ;
(b) des polynucléotides ayant la séquence codante telle que décrite dans SEQ ID n° 2, codant pour au moins la forme mature du polypeptide ;
(c) des polynucléotides codant pour le polypeptide ayant la séquence d'aminoacides d'au moins la forme mature du polypeptide codé par fADNc contenu dans ATCC 75927 ;
(d) des polynucléotides ayant la séquence de l'ADNc contenu dans ATCC 75927 codant pour au moins la forme mature du polypeptide ; et
(e) des polynucléotides ayant une identité d'au moins 95 % avec un polynucléotide de l'un quelconque des points (a) à (d), qui codent pour un polypeptide stimulant la libération de cytokines pro-inflammatoires par les lymphocytes T.

2. Polynucléotide selon la revendication 1, qui est un ADN.

3. Polynucléotide selon la revendication 2, qui est un ADN génomique.

4. Polynucléotide selon la revendication 1, qui est un ARN.

5. Vecteur contenant le polynucléotide selon l'une quelconque des revendications 1 à 4.

6. Vecteur selon la revendication 5, dans lequel le polynucléotide est lié de façon opérationnelle à des séquences régulatrices d'expression permettant l'expression dans des cellules hôtes procaryotes ou eucaryotes.

7. Cellule hôte génétiquement modifiée par le vecteur selon la revendication 5 ou 6.

8. Procédé pour la production d'un polypeptide ayant une activité de TNF-gamma, comprenant la culture de la cellule hôte selon la revendication 8 et la récupération du polypeptide codé par ledit polynucléotide à partir de la culture.

9. Procédé pour la production de cellules capables d'exprimer un polypeptide ayant une activité de TNF-gamma, comprenant la modification génétique de cellules par le vecteur selon la revendication 5 ou 6.

10. Polypeptide ayant la séquence d'aminoacides codée par un polynucléotide selon l'une quelconque des revendications 1 à 4 ou pouvant être obtenu par un procédé selon la revendication 8.

11. Anticorps dirigé contre le polypeptide selon la revendication 10.

12. Antagoniste/inhibiteur contre le polypeptide selon la revendication 10, qui est un anticorps selon la revendication 11 ou un produit de construction antisens qui est capable de s'hybrider avec un transcrit du polynucléotide selon l'une quelconque des revendications 1 à 4.

13. Molécule d'acide nucléique qui se lie spécifiquement à un polynucléotide selon l'une quelconque des revendications 1 à 4.

14. Composition pharmaceutique, comprenant le polynucléotide selon l'une quelconque des revendications 1 à 4 ou le polypeptide selon la revendication 10 et éventuellement un véhicule pharmaceutiquement acceptable.

15. Composition de diagnostic, comprenant le polynucléotide selon l'une quelconque des revendications 1 à 4, la molécule d'acide nucléique selon la revendication 13 ou l'anticorps selon la revendication 11.

16. Utilisation du polypeptide selon la revendication 10 ou du polynucléotide selon l'une quelconque des revendications 1 à 4, pour la préparation d'une composition pharmaceutique destinée à la cicatrisation, à l'inhibition de la néoplasie ou de la prolifération de tumeurs, au traitement de la resténose ou à la prévention de maladies auto-immunes.

17. Utilisation de l'antagoniste selon la revendication 12, pour la préparation d'une composition pharmaceutique destinée au traitement de la cachexie, du paludisme cérébral, de la polyarthrite rhumatoïde, de l'ostéoporose, du choc endotoxique (septique), à la prévention du rejet de greffe ou utilisable en tant qu'agent anti-inflammatoire.

18. Procédé pour l'identification d'agonistes et d'antagonistes du polypeptide selon la revendication 10, comprenant :
(a) la combinaison de cellules endothéliales, de concanavaline A, du composé à analyser, de [³H]-thymidine sélectivement en présence ou en absence du polypeptide de la revendication 10 ;
(b) la mesure de l'incorporation de [³H]-thymidine par les cellules endothéliales ; et
(c) le fait de déterminer si le composé a augmenté ou bloqué l'incorporation de [³H]-thymidine.

19. Procédé in vitro pour diagnostiquer une tumeur ou une sensibilité à une tumeur, comprenant la détection d'une forme mutée de la séquence d'acide nucléique codant pour le polypeptide selon la revendication 10.

20. Anticorps selon la revendication 11, qui est un anticorps polyclonal.

21. Anticorps selon la revendication 11, qui choisi dans le groupe constitué par :
(a) un anticorps monoclonal ;
(b) un anticorps chimérique ;
(c) un anticorps monocaténaire ; et
(d) un fragment Fab.

22. Cellule produisant l'anticorps selon la revendication 11.

23. Hybridome produisant l'anticorps selon la revendication 11.
